# EUROPEAN PATENT SPECIFICATION

(11) **EP 3 826 587 B1**
(45) Date of publication and mention of the grant of the patent: **15.04.2026**
(21) Application number: 19756047.7
(22) Date of filing: 23.07.2019
(51) Int. Cl.: A61F 2/07, A61F 2/06

(54) **IMPLANTABLE MEDICAL DEVICES FOR FLUID FLOW CONTROL**
IMPLANTIERBARE MEDIZINISCHE VORRICHTUNGEN ZUR FLÜSSIGKEITSSTRÖMUNGSREGELUNG
DISPOSITIFS MÉDICAUX IMPLANTABLES POUR RÉGULER LE FLUX DE FLUIDE

(30) Priority: 24.07.2018 US 201862702652 P; 24.07.2018 US 201862702666 P; 24.07.2018 US 201862702677 P
(43) Date of publication of application: 02.06.2021
(73) Proprietor: W. L. Gore & Associates, Inc., Newark, DE 19711 (US)
(72) Inventor: BRINKMANN, John M., Newark, Delaware 19711 (US); COFFMAN, Kevin K., Newark, Delaware 19711 (US); CULLY, Edward H., Newark, Delaware 19711 (US); DUGDALE, Joel W., Newark, Delaware 19711 (US); DUNCAN, Jeffrey B., Newark, Delaware 19711 (US); GOEPFRICH, James L., Newark, Delaware 19711 (US); GOODMAN, Paul D., Newark, Delaware 19711 (US); JOHNSON, Dorthyann I., Newark, Delaware 19711 (US); MCDANIEL, Tom R., Newark, Delaware 19711 (US); MOKELKE, Eric A., Newark, Delaware 19711 (US); SHAW, Edward E., Newark, Delaware 19711 (US); TROKANSKI, Mark E., Newark, Delaware 19711 (US); VONESH, Michael J., Newark, Delaware 19711 (US)
(74) Representative: HGF
(86) International application number: PCT/US2019/043045
(87) International publication number: WO 2020/023514

(56) References cited:
- WO-A1-2015/006607
- US-A1- 2003 236 567
- US-A1- 2008 051 879

## Description

### TECHNICAL FIELD

The present disclosure relates to systems, medical devices, and methods for treating heart failure and/or other cardiovascular diseases. More specifically, the disclosure relates to removing buildup of excess fluid that typically results from under-perfused kidneys.

### BACKGROUND

Patients experiencing heart failure may have a buildup of excess fluid in the body. The excess fluid buildup may increase fluid accumulation in the interstitial space and worsen a patient's symptoms and quality of life. Excess fluid (or hypervolemia) is the leading cause of hospitalization for heart failure patients (approximately 1,000,000 per year in the United States).

Treatment of the excess fluid buildup may be treated pharmaceutically by diuretics (or other pharmaceutical agents). However, a patient may experience drug resistance, unwanted side effects, inappropriate dosing, or other issues such as failure to comply with medicine directives. Non-pharmaceutical options, such as implantable device solutions that provide an alternative to or augment pharmaceutical efficacy by influencing renal function, may be beneficial to avoid these and other issues in treatment of buildup of excess fluid in the body. Similarly, chronic high blood pressure (hypertension) can also be managed pharmaceutically by diuretics (or other antihypertensive pharmaceutical agents). In addition, other disease states may result in hypotension, reduced cardiac output, and poor renal function. Insofar as the kidneys play a central role in regulating systemic blood pressure and fluid homeostasis, non-pharmaceutical options, such as implantable device solutions that provide an alternative to or augment pharmaceutical efficacy by influencing renal function, may provide an alternative means of managing the fluid imbalance resulting from chronic disease states such as heart failure, hypertension and other disease states. Such implantable devices are disclosed in the documents US2003/236567A1, WO2015/006607A1 and US2007/293808A1.

### SUMMARY

The invention is in accordance with claim 1. Selected further features are set out in the dependent claims.

While multiple examples are disclosed herein, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 shows an example implantable medical device in accordance with various aspects of the present disclosure.
FIG. 2 shows an example implantable medical device having branches.
FIG. 3A shows a side view of an example implantable medical device having a restricting portion and an anchor portion.
FIG. 3B shows an end view of the implantable medical device shown in FIG. 3A.
FIG. 4A shows a side view of another example implantable medical device having a restricting portion and an anchor portion.
FIG. 4B shows the implantable medical device shown in FIG. 4A implanted in a vascular of a.
FIG. 5 shows a side view of an example implantable medical device having a restricting portion and an adjustable portion.
FIGS. 6A-6B show a side view of another example implantable medical device having a restricting portion and an adjustable portion in accordance with various aspects of the present disclosure.
FIGS. 7A-7C show a side view of another example implantable medical device having a restricting portion and an adjustable portion in accordance with various aspects of the present disclosure.
FIG. 8 shows an example implantable medical device including a body portion and an adjustable portion.
FIG. 9A shows an example implantable medical device including a body portion and an adjustable portion in a first configuration.
FIG. 9B shows the example implantable medical device, shown in FIG. 9A, in a second configuration.
FIG. 9C shows the example implantable medical device, shown in FIGS. 9A-B.
FIG. 9D shows the example implantable medical device, shown in FIGS. 9A-C, in a fourth configuration.
FIG. 10 shows another example implantable medical device.
FIG. 11A shows another example implantable medical device in a first configuration.
FIG. 11B shows the implantable medical device, shown in FIG. 11A, in a second configuration.
FIG. 12A shows an example adjustable portion of an implantable medical device in a first configuration.
FIG. 12B shows the adjustable portion, shown in FIG. 12A, in a second configuration.
FIG. 12C shows the adjustable portion, shown in FIGs. 12A-12B, in a third configuration.
FIG. 13A shows another example adjustable portion of an implantable medical device in a first configuration.
FIG. 13B shows the adjustable portion, shown in FIG. 13A, in a second configuration.
FIG. 13C shows the adjustable portion, shown in FIGs. 13A-13B, in a third configuration.
FIG. 14A-14C shows an example implantable medical device including a first body portion, a second body portion, and an adjustable portion in various configurations.
FIG. 15 shows an example implantable medical device including a body portion and an adjustable portion about an external surface of the body portion.
FIG. 16A-16B shows an example implantable medical device including an adjustable portion in various configurations.
FIG. 17 shows an example implantable medical device and an expandable device.
FIG. 18 shows an example vacuum for adjusting blood flow.
FIG. 19 shows an example implantable medical device.
FIG. 20 shows an example implantable restriction device.
FIG. 21 shows an example implantable medical device having branches.
FIG. 22 shows another example implantable medical device having branches arranged within a vessel and side branch vessels.
FIG. 23A-23D show various example implantable medical devices.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

Certain terminology is used herein for convenience only. For example, words such as "top", "bottom", "upper," "lower," "left," "right," "horizontal," "vertical," "upward," and "downward" merely describe the configuration shown in the figures or the orientation of a part in the installed position. Indeed, the referenced components may be oriented in any direction. Similarly, throughout this disclosure, where a process or method is shown or described, the method may be performed in any order or simultaneously, unless it is clear from the context that the method depends on certain actions being performed first.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward treating heart failure in a patient and/or other cardiovascular diseases such as hypertension and hypotension. In certain instances, the condition of the patient may deteriorate by buildup of excess fluid (*e.g*., hypervolemia) in the body. The buildup of fluid may increase fluid accumulation, principally in the tissues, and increase fluid and pressure in the various circulations and organs. The increased fluid and pressure in and of itself or in combination with an already failing heart may further harm the patient. As discussed in further detail below, various aspects of the present discourse are directed toward lessening buildup of excess fluid by use of an implantable medical device.

Various aspects of the disclosure are directed toward an implantable medical device configured to manipulate renal blood flow hemodynamics in order to induce a physiologically mediated therapeutic response. The implantable medical device discussed herein, in certain instances, is intended to increase natural diuresis and lessen buildup of excess fluid by increasing blood flow to the kidneys. By this action, this device is configured to redirect blood flow to the kidneys to reperfuse the kidneys, improve diuresis (increase fluid removal) and minimize/eliminate the impact of fluid overload on the heart. Kidney health may include the amount of injury that the kidney has sustained, is continuing to sustain, or a decrease in function relative to the baseline kidney function of a patient when healthy. In certain instances, kidney injury may be quantified by measuring Neutrophil gelatinase-associated lipocalin (NGAL).

Also disclosed are methods that include arranging a flow restriction device within the aorta or vena cava of a patient. When a flow restriction device is arranged within the aorta, the device may also be configured to alter blood flow into at least one branch vessel of the aorta while maintaining a substantially unrestricted blood flow within the aorta proximal to the branch vessel. In instances where a flow restriction device is arranged within the vena cava, the device may also be configured to increase blood flow from at least one tributary vessel of the vena cava.

When implanted in the aorta, the flow restriction devices are configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the flow restriction devices are configured to create a narrowed flow lumen in the conduit located in the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the flow restriction device may augment perfusion from a tributary vessel (*e.g*., renal veins) terminating in the vena cava by altering pressure within the vena cava to alter blood flow from the tributary vessel of the vena cava. In certain instances, the flow restriction device may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

In certain instances, the flow restriction devices discussed herein may be implanted in other vessels. The flow restriction devices may facilitate increase in peripheral resistance to treat decreases in blood pressure or resistance within the vasculature. As discussed further below, this may include implantation of the flow restriction devices for treatment of an arteriovenous (AV) fistula.

In certain instances, the flow restriction devices may be arranged in a vessel other than the aorta or venal cava. In these instances, the flow restriction devices may be configured to alter the blood flow through the lumen of the device to restrict blood flow in the vessel and induce a physiologically mediated therapeutic response in the patient. In certain instances, the flow restriction devices are configured to induce the physiologically mediated therapeutic response to include an increase in peripheral resistance within the vessel. The flow restriction devices may be configured to treat a fistula within the vessel and increase in peripheral resistance within the vessel as described in further detail below.

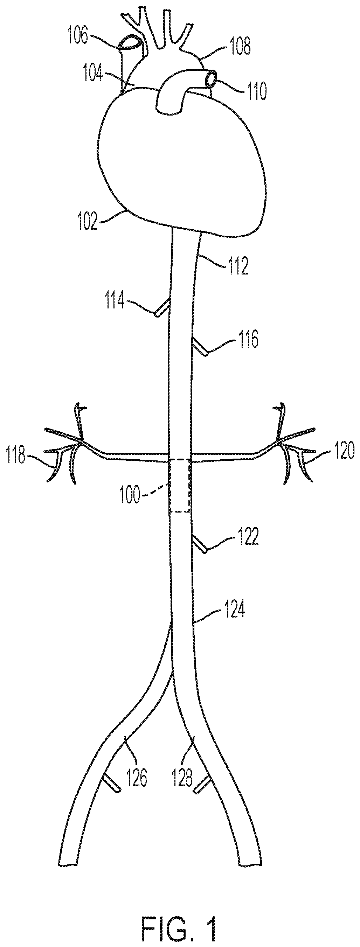

FIG. 1 shows an example implantable medical device 100 in accordance with various aspects of the present disclosure. The implantable medical device 100 is shown arranged within a patient's vasculature. The patient's vasculature shown in FIG. 1 includes the patient's heart 102, aortic root 104, superior vena cava 106, aortic arch 108, pulmonary trunk 110, descending aorta 112, celiac artery 114, superior mesenteric artery 116, renal arteries 118, 120, inferior mesenteric artery 122, abdominal aorta 124, and iliac arteries 126, 128. The implantable medical device 100 may be arranged within the aorta distal of the renal arteries 118, 120. In addition, the implantable medical device 100 may be configured to increase blood flow into at least one of the renal arteries 118, 120 while maintaining a substantially unrestricted blood flow within the aorta proximal to the renal arteries 118, 120. In addition, the implantable medical device 100 may be arranged within one or both of the iliac arteries 126, 128 in addition to or alternatively of the implantable medical device 100 being arranged within the aorta distal to the renal arteries 118, 120.

In certain instances, the implantable medical device 100 may be for augmenting perfusion of a branch vessel (*e.g*., renal arteries 118, 120 or iliac arteries 126, 128) originating from the aorta. The implantable medical device 100 may be adjusted by increasing resistance to blood flow through the implantable medical device 100 to increase pressure within the aorta to increase blood flow into the branch vessel. In addition, the implantable medical device 100 may be configured to remain within the aorta for continuously augmenting perfusion.

The implantable medical device 100 being configured to increase blood flow into at least one of the renal arteries 118, 120 may reduce fluid accumulation by increasing the amount of blood that is filtered by the kidneys. In a patient suffering from heart failure, fluid overload may be caused (at least in part) by insufficient blood flow through the kidneys resulting from compromised cardiac output and venous congestion. Use of the implantable medical device 100 to increase blood flow into at least one of the renal arteries 118, 120 may increase kidney perfusion hemodynamically rather than pharmaceutically. To achieve increased kidney perfusion, resistance to blood flow distal to the renal arteries 118, 120 may be increased, which may decrease distal perfusion. The increased kidney perfusion enhances renal filtration and therefore removes fluid volume. In addition, the implantable medical device 100 may be used to enhance the performance of pharmacological treatments taken in connection therewith. For example, pharmacological treatments (*e.g*., diuretics and/or hypertensive medications) may be enhanced by additionally enhancing the patient's kidney function.

In certain instances, the implantable medical device 100 being configured to increase blood flow into at least one of the renal arteries 118, 120 while maintaining a substantially unrestricted blood flow within the aorta proximal to the renal arteries 118, 120 may focus blood flow into the one or both of the renal arteries 118, 120. The restriction proximal to the renal arteries 118, 120 may direct blood flow to other areas supplied by the aorta such as the celiac artery 114, the superior mesenteric artery 116, or the brain. Thus, in certain instances, the implantable medical device 100 may be arranged within the aorta of the patient distal of (or overlapping) the renal arteries 118, 120. The result may be increased blood flow to at least one of the kidneys, by way of the increased blood flow to one or both of the renal arteries 118, 120, which may increase fluid removal from the circulation which would relieve the fluid and pressure accumulation in the various circulations and organs.

The implantable medical device 100 provides a non-pharmaceutical approach to increasing urine production (diuresis) and/or modifying systemic blood pressure. Patients may experience drug resistance, inaccurate dosing, or undesirable side effects. When drugs fail, aquapheresis or hemodialysis may be used to filter fluid directly from blood, however, these solutions are relatively invasive and disruptive to patient lifestyle and mobility. In addition, aquapheresis or hemodialysis may also produce hemodynamic instability with related cardiovascular complications, kidney damage, infection, and/or require capital equipment.

The implantable medical device 100 may change peripheral resistance when implanted percutaneously or surgically, temporarily or permanently, and may be adjustable to meet patient needs. The implantable medical device 100 may remain in the body after implantation for as long as the patient requires intervention. The implantable medical device 100 may be implanted for hours, days, or even years.

The illustrative implantable medical device 100 shown in FIG. 1 is not intended to suggest any limitation as to the scope of use or functionality of embodiments of the disclosure disclosed throughout this document. Neither should the illustrative implantable medical device 100 be interpreted as having any dependency or requirement related to any single component or combination of components illustrated therein. Additionally, any one or more of the components depicted in FIG. 1 can be, in embodiments, integrated with various ones of the other components depicted therein (and/or components not illustrated).

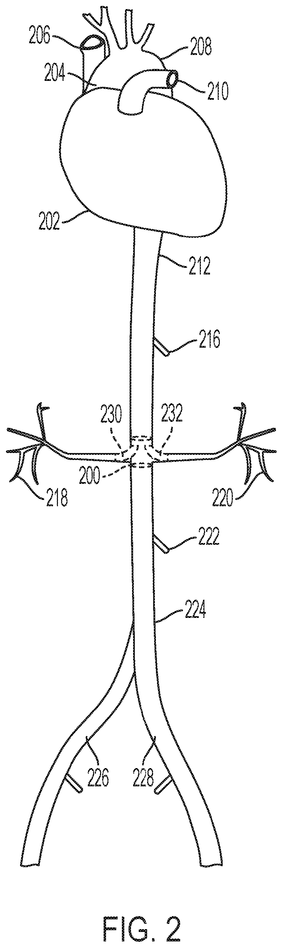

FIG. 2 shows an example implantable medical device 200 having branches 230, 232 (not in accordance with claim 1) in accordance with various aspects of the present disclosure. The implantable medical device 200 is shown arranged with a patient's aorta. The patient's vasculature shown in FIG. 2 includes the patient's heart 202, aortic root 204, superior vena cava 206, aortic arch 208, pulmonary trunk 210, descending aorta 212, celiac artery 212, superior mesenteric artery 216, renal arteries 218, 220, inferior mesenteric artery 222, abdominal aorta 224, and iliac arteries 226, 228. The implantable medical device 200 may be arranged with the aorta distal of the renal arteries 218, 220.

The implantable medical device 200 may be configured to increase blood flow into at least one of the renal arteries 218, 220. The implantable medical device 200, which may be formed of a graft and stent combination, may include one or more branches 230, 232. The branches 230, 232 may be arranged within the renal arteries 218, 220, with a main body portion of the implantable medical device 200 being arranged within the aorta. The branches 230, 232 of the implantable medical device 200 may direct blood flow into the renal arteries 218, 220 to increase blood flow therein. The implantable medical device 200 allows for blood flow distally to the implantable medical device 200. The branches 230, 232 of the implantable medical device 200 may increase resistance for blood flow distally of the implantable medical device 200 thereby increasing blood into the renal arteries 218, 220.

In certain instances, the implantable medical device 200 may be diametrically adjustable. In certain instances, the implantable medical device 200 may be diametrically adjustable by way of a distensible force applied within the implantable medical device 200. The force may be applied within the main body portion of the implantable medical device 200 or within the branches 230, 232 to adjust the dimensions. Dimensions of the branches 230, 232 or other portions of the implantable medical device 200 may be restricted or expanded to adjust flow. The implantable medical device 200 may be fabricated of Nitinol (NiTi) or stainless steel and ePTFE, and may be self-expanding or balloon expandable.

The implantable medical device 200 may also maintain a substantially unrestricted blood flow within the aorta proximal to the renal arteries 218, 220. Blood flow may be monitored proximal to the renal arteries 218, 220 and the amount of restriction applied via the implantable medical device 200 may be adjusted to maintain a substantially normal flow to areas of the aorta proximal to the renal arteries 218, 220 and also to increase blood flow into at least one of the renal arteries 218, 220.

When implanted in the aorta, the device 200 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 200 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 200 may augment perfusion from a tributary vessel (*e.g*., renal veins) terminating in the vena cava by altering pressure within the vena cava to alter blood flow from the tributary vessel of the vena cava. In certain instances, the device 200 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

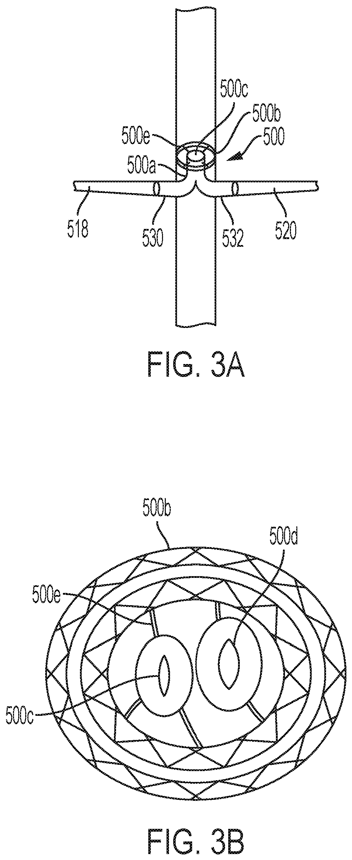

FIG. 3A shows a side view of an example implantable medical device having a restricting portion and an anchor portion (not according to claim 1). FIG. 3A shows an example fluid directing device 500 that includes a restricting portion 500a and an anchor portion 500b. The device 500 is shown arranged with a patient's vessel such as the patient's aorta. For example, the device 500 may be arranged with the vessel distal of the branch vessels 518, 520 (*e.g*., renal, celiac, the hepatic, or mesenteric arteries).

In certain instances, the device 500 includes the restricting portion 500a and the anchor portion 500b. The anchor portion 500b may be a structure capable of maintaining the device 500 within the patient's vessel (aorta) via a pressure fit. For example, the anchor portion 500b may be a ring-like structure having approximately the same or slightly greater diameter than the diameter of the patient's aorta. The restricting portion 500a is connected to the anchor portion 500b by one or more attachment arms 500e. As shown, the restricting portion 500a may be suspended in the center of the anchor portion 500b or at any location within the anchor portion 500b as desired. Though not shown in FIG. 3A, in some instances, the device 500 may be maintained in the patient's aorta via anchors or other suitable retention devices.

The device 500 may be configured to increase blood flow into at least one of the branch vessels 518, 520. The implantable medical device 500 may include one or more branches 530, 532. The branches 530, 532 may be arranged within the branch vessels 518, 520, with a main body portion of the implantable medical device 500 being arranged within the aorta. The branches 530, 532 of the implantable medical device 500 may direct blood flow into the branch vessels 518, 520 to increase blood flow therein. The implantable medical device 500 allows for blood flow distally to the implantable medical device 500. In addition, the branches 530, 532 of the implantable medical device 500 may increase resistance for blood flow distally of the implantable medical device 500 thereby increasing blood into the branch vessels 518, 520.

In certain instances, the restricting portion 500a includes a first fluid inlet 500c. The first inlet portion 500c splits into the one or more branches 530, 532 having first and second fluid outlets 540, 542, as shown. Thus, the restricting portion 500a directs fluid from the aorta through the first fluid inlet 500c, into the one or more side branches 530, 532, and out the first and second fluid outlets 540, 542 into the renal arteries.

In certain instances, the restricting portion 500b includes first and second fluid inlets 500c, 500d as shown in FIG. 3B. The first and second fluid inlets 500c, 500d may be arranged adjacent one another within the anchor portion 500b. Each of the first and second fluid inlets 500c, 500d may lead into the one or more side branches 530, 532. For example, the first inlet portion 500c may lead into a first side branch 530 and the second inlet portion 500d may lead into a second side branch 532.

In certain instances, the anchor portion 500b is configured to arrange the inlet(s) 500c, 500d of the restricting portion 500a at a maximum fluid velocity section of the blood vessel. The stent and the graft component, of the restricting portion 500a, form an inlet 500c or inlets 500c, 500d arranged approximately centrally within the vessel to displace blood from the portion of the vessel having higher blood flow velocity to increase blood flow into one or both of the side branches 530, 532. The restricting portion 500a and the anchor portion 500b manipulate the velocity profile of blood within a vessel to direct blood to one or more side branches 530, 532. In addition, the stent element and the graft component, of the restricting portion 500a and the anchor portion 500b, are configured to allow fluid flow within the vessel between the restricting portion 500a and the anchor portion 500b. The restricting portion 500a and the anchor portion 500b may induce resistance to blood flow within the vessel and thereby increase blood flow into one or both of the side branches 530, 532 through the manipulation of blood flow as described above. In certain instances, the restricting portion 500a and the anchor portion 500b are configured to induce stenosis of the vessel (aorta) distal of the at least one side branches 530, 532 between about 40% and about 80% to improve kidney perfusion and diuresis.

In certain instances, the restricting portion 500a and the anchor portion 500b may decrease a blood flow rate, within the vessel (aorta) distal to the side branches 518, 520 (*e.g.,* renal arteries), by between about 5% and about 30% as compared to normal flow. The restricting portion 500a and the anchor portion 500b may occlude the aorta distal to the side branches 518, 520 (*e.g.,* renal arteries) by between about 5% and about 30% to increase blood flow into the kidneys. In certain instances, the restricting portion 500a and the anchor portion 500b are configured to induce stenosis of the aorta of the patient distal of the side branches 518, 520 (*e.g.,* renal arteries) between about 40% and about 80% and alter blood flow into one or more of the side branches 518, 520 (*e.g.,* renal arteries) while maintaining a substantially unrestricted blood flow within the vessel (aorta) 200 proximal to one or more of the side branches 518, 520 (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

In addition, the restricting portion 500a and the anchor portion 500b may be implanted into another vessel of the patient that leads into an organ. In these instances, the restricting portion 500a and the anchor portion 500b is configured to induce stenosis of the vessel into which the restricting portion 500a and the anchor portion 500b distal of location at which the restricting portion 500a and the anchor portion 500b is implanted between about 40% and about 80%. In addition, implanting the restricting portion 500a and the anchor portion 500b in this manner alter blood flow into the organ that the vessel leads into while maintaining a substantially unrestricted blood flow within the vessel proximal to the location of implantation.

In certain instances, the restricting portion 500a and the anchor portion 500b are configured to increase blood pressure at an ostium of the at least one of the renal artery (one or both side branches 530, 532) relative to venous outflow pressure causing more blood to flow through the kidney. In these instances, increasing the blood pressure at the ostium of the at least one renal artery (one or both side branches 530, 532) includes increasing fluid filtration by the kidney to increase diuresis and lessen fluid retention of the patient.

When implanted in the aorta, the device 500 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 500 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 500 may augment perfusion from a tributary vessel (*e.g.,* renal veins) terminating in the vena cava by altering pressure within the vena cava to alter blood flow from the tributary vessel of the vena cava. In certain instances, the device 500 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

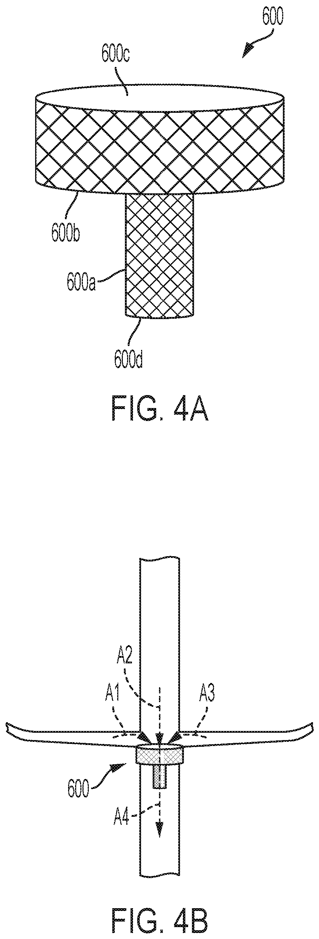

FIG. 4A shows an example implantable medical device 600 that includes a restricting portion 600a and an anchor portion 600b (not according to claim 1). As shown, the anchor portion 600b may be a structure capable of maintaining the device 600 in the patient's vessel via a pressure fit. For example, the anchor portion 600b may be a ring-like structure having approximately the same or slightly greater diameter than the diameter of the patient's vessel. Though not shown in FIG. 4A, in some instances, the device 600 may be maintained in the patient's aorta via anchors or other suitable retention devices.

In certain instances, the anchor portion 600b may be diametrically adjustable. In certain instances, the anchor portion 600b may be diametrically adjustable by way of a distensible force applied within the device 600. For example, the device 600 may be fabricated of Nitinol (NiTi) or stainless steel and ePTFE, and may be self-expanding or balloon expandable.

The restricting portion 600a is attached to the anchor portion 600b. As shown, the restricting portion 600a may have a smaller diameter than the anchor portion 600b to facilitate restriction of blood flow through the device and increased fluid flow to desired areas of the body and/or a broad range of fluid flow through the device. For example, a ratio of the diameter of the restricting portion 600a to the anchor portion 600b may be from about 0.2 to about 0.8, from about 0.3 to about 0.7, or from about 0.4 to about 0.6, and will depend on the desired fluid flow rate through the device 600.

FIG. 4B shows the implantable medical device shown in FIG. 4A implanted in a vascular of a patient. The device 600 is shown arranged with a patient's aorta. The device 600 may be arranged with the aorta distal of the renal arteries 618, 620 (not shown), for example. In addition, the implantable medical device 600 may be configured to increase blood flow into at least one of the renal arteries 618, 620 while maintaining a substantially unrestricted blood flow within the aorta proximal to the renal arteries 618, 620. In addition, the implantable medical device 600 may be arranged around one or both of the iliac arteries (not shown) in addition to or alternatively of the implantable medical device 600 being arranged with the aorta distal of the renal arteries 618, 620.

Arrows A1, A2, and A3 show a direction of fluid flow into the device 600 from the aorta and renal arteries 618, 620. Fluid flows from the aorta and renal arteries 618, 620 into an inlet portion 600c of the device. The change in diameter between the inlet portion 600c and anchor portion 600b may increase the rate of fluid flow from the renal arteries 618, 620, for example, or may generally redirect flow as desired. Arrow A4 shows a direction of fluid flow out of the device 600 through the outlet portion 600d.

In certain instances, the anchor portion 600b is configured to arrange the inlet portion 600c at a maximum fluid velocity section of the blood vessel. The inlet portion 600c may be arranged approximately centrally within the vessel to displace blood from the portion of the vessel having higher blood flow velocity to increase blood flow into one or both of the side branches.

The restricting portion 600a may induce resistance to blood flow within the vessel and thereby increase blood flow into one or both of the side branches through the manipulation of blood flow as described above. In certain instances, the restricting portion 600a are configured to induce stenosis of the vessel (aorta) distal of the at least one side branches between about 40% and about 80% to improve kidney perfusion and diuresis.

In certain instances, the restricting portion 600a may decrease a blood flow rate, within the vessel (aorta) distal to the side branches (e.g., renal arteries), by between about 5% and about 30% as compared to normal flow. The restricting portion 600a may occlude the aorta distal to the side branches (*e.g.,* renal arteries) by between about 5% and about 30% to increase blood flow into the kidneys. In certain instances, the restricting portion 600a are configured to induce stenosis of the aorta of the patient distal of the side branches (*e.g.,* renal arteries) between about 40% and about 80% and alter blood flow into one or more of the side branches (*e.g.,* renal arteries) while maintaining a substantially unrestricted blood flow within the vessel (aorta) 200 (FIG. 2) proximal to one or more of the side branches 618, 620 (e.g., renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

In addition, the restricting portion 600a and the anchor portion 600b may be implanted into another vessel of the patient that leads into an organ. In these instances, the restricting portion 600a and the anchor portion 600b is configured to induce stenosis of the vessel into which the restricting portion 600a and the anchor portion 600b distal of location at which the restricting portion 600a and the anchor portion 600b is implanted between about 40% and about 80%. In addition, implanting the restricting portion 600a and the anchor portion 600b in this manner alter blood flow into the organ that the vessel leads into while maintaining a substantially unrestricted blood flow within the vessel proximal to the location of implantation.

As shown in FIG. 4A-B, the restricting portion 600a includes a reduced diameter section, relative to the anchor portion 600b to induce stenosis of the vessel distal of the at least one branch vessel of the aorta between about 50% and about 70%. In addition, the restricting portion 600a (the reduced diameter section) is substantially cylindrical.

When implanted in the aorta, the device 600 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 600 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 600 may augment perfusion from a tributary vessel (*e.g.,* renal veins) terminating in the vena cava by altering pressure within the vena cava to alter blood flow from the tributary vessel of the vena cava. In certain instances, the device 600 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

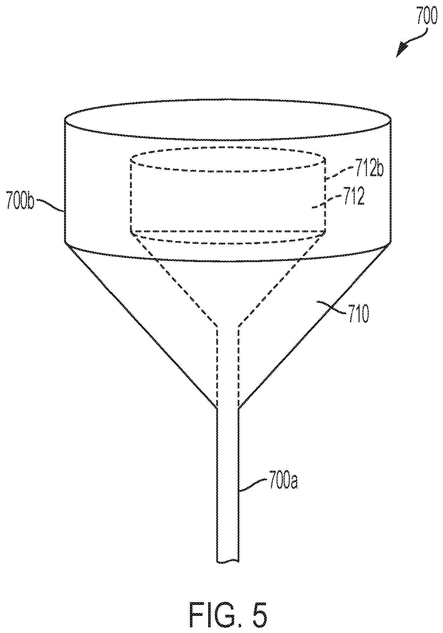

FIG. 5 shows an example implantable medical device 700 that includes a restricting portion 700a and an adjustable portion 700b (not according to claim 1). In certain instances, the device 700 may be arranged with a patient's aorta. For example, the device 700 may be arranged with the aorta distal of the renal arteries (not shown).

As shown, the adjustable portion 700b may be a structure capable of maintaining the device 700 in the patient's aorta via a pressure fit. For example, the adjustable portion 700b may be a ring-like structure having approximately the same or slightly greater diameter than the diameter of the patient's aorta. In some examples, the adjustable portion 700b may be a stent ring made of nitinol or other suitable biocompatible materials, or may be a cylindrical or cone-like structure. Though not shown in FIG. 5, in some instances, the device 700 may be maintained in the patient's aorta via anchors or other suitable retention devices.

The restricting portion 700a is attached to the adjustable portion 700b. As shown, the restricting portion 700a may have a smaller diameter than the adjustable portion 700b to facilitate restriction of blood flow through the device and increased or decreased fluid flow to desired areas of the body. For example, a ratio of the diameter of the restricting portion 700a to the adjustable portion 700b may be from about 0.2 to about 0.8, from about 0.3 to about 0.7, or from about 0.4 to about 0.6, and will depend on the desired fluid flow rate through the device 700. In certain instances, the device 700 may incrementally decrease in diameter from the adjustable portion 700b to the restricting portion 700a. For example, the device 700 may be tapered to facilitate fluid flow therethrough.

In certain instances, the adjustable portion 700b may have an outer structure 710 and an inner structure 712. The inner structure 712 may be nested inside the outer structure 710, as shown in FIG. 5. The inner structure 712 may be connected to the restricting portion 700a. For example, the restricting portion 700a may be connected to the inner structure 712 and may extend through the outer structure 710, as shown. In certain instances, the inner structure 712 may be identical in shape to the outer structure 710. For example, both the outer and inner structures 710, 712 may be cylindrical, conical, tapered, or any other suitable shape as desired. In certain instances, the outer structure 710 and the inner structure 712 are separate components, and in other instances, the outer structure 710 and the inner structure 712 a formed of a single integral structure. In instances where the outer structure 710 and the inner structure 712 are separate components, the outer structure 710 may be delivered first and the inner structure 712 delivered second and adjusted upon delivery to achieve a desired flow.

As shown, the inner structure 712 is smaller than the outer structure 710. For example, a ratio of a diameter of an inner anchor portion 712b of the inner structure 712 to the adjustable portion 700b of the outer structure 710 may be from about 0.2 to 0.8, from about 0.3 to 0.7, or from about 0.4 to 0.6 and will depend on the desired fluid flow rate through the device 700.

In some embodiments, the inner structure 712 is rotatable with respect to the outer structure 710. For example, rotating the inner structure 712 with respect to the outer structure 710 may adjust a diameter of the restricting portion 700a respective to the amount of rotation. For example, rotating the inner structure 712 with respect to the outer structure 710 may decrease the diameter of the restricting portion 700a by between about 10% and about 90% or fully occlude the restricting portion 700a. in certain instances, each of the inner structure 712 with respect to the outer structure 710 include an outlet and the directed blood flow increases in response to alignment of the outlet of the inner structure 712 and the outlet of the outer structure 710 and decreases in response to misalignment of the outlet of the inner structure 712 and the outlet of the outer structure 710. In certain instances, the adjustable portion 700b may be necked down or elongated to further apply restriction. In other instances, the adjustable portion 700b may be balloon expandable.

As described in detail above, the adjustable portion 700b, including the inner structure 712 (a first adjustable section) and the outer structure 710 (a second adjustable section), includes one or both of the adjustable section being configured to rotate to alter the amount of directed blood flow by the restricting portion 700a. In certain instances, the adjustable portion 700b may increase or decrease a blood flow rate, within the vessel (aorta) distal to the side branches (*e.g.,* renal arteries), by between about 5% and about 30% as compared to normal flow. The adjustable portion 700b may occlude the aorta distal to the side branches (*e.g.,* renal arteries) by between about 5% and about 30% to increase blood flow into the kidneys. In certain instances, the fluid adjustable portion 700b is configured to induce stenosis of the aorta of the patient distal of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 700 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 700 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 700 may augment perfusion from a tributary vessel (*e.g.,* renal veins) terminating in the vena cava by altering pressure within the vena cava to alter blood flow from the tributary vessel of the vena cava. In certain instances, the device 700 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

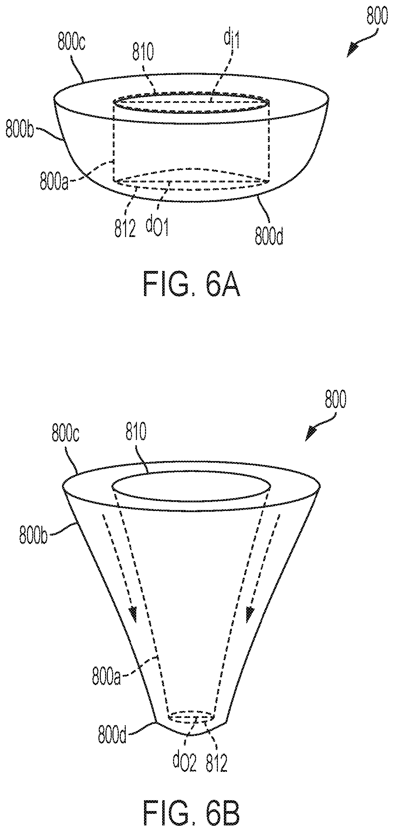

FIGS. 6A-6B show an example implantable medical device 800 that includes a restricting portion 800a and an adjustable portion 800b in accordance with various aspects of the present disclosure. In certain instances, the device 800 may be arranged with a patient's aorta. For example, the device 800 may be arranged with the aorta distal of the renal arteries (not shown).

In certain instances, the device 800 may be, for example, an implantable, expandable stent structure. The device 800 may have multiple layers, including the adjustable portion 800b and the restricting portion 800a, for example. In certain instances, the restricting portion 800a may be, for example, a membrane having central orifices to allow for fluid flow therethrough. For example, the restricting portion 800a may form a channel through the adjustable portion 800b to allow fluid flow therethrough.

FIG. 6A shows the device 800 in a relaxed configuration. As shown, the adjustable portion 800b may be substantially conical or tapered in shape. For example, a first end 800c of the device 800 may have a larger diameter than a second end 800d of the device 800. As shown, the restricting portion 800a can be located in the center of the adjustable portion 800b. The restricting portion 800a forms a channel through the adjustable portion 800b to allow fluid flow therethrough (e.g., from the first end 800c to the second end 800d).

The restricting portion 800a includes a fluid inlet 810 and a fluid outlet 812. When the device 800 is in the relaxed configuration, the fluid inlet 810 has a first inlet diameter dᵢ₁ and the fluid outlet 812 has a first outlet diameter d_{O1}. As shown, the first inlet and outlet diameters dᵢ₁, d_{O1} may be approximately the same when the device 800 is in the relaxed configuration. For example, a ratio of the first inlet diameter dᵢ₁ to the first outlet diameter d_{O1} may be about 1 to 1 when the device 800 is in the relaxed configuration.

FIG. 6B shows the device 800 in a stretched configuration. As shown, as the device 800 lengthens from the relaxed configuration to the stretched configuration, the fluid outlet 812 changes from the first outlet diameter d_{O1} to a second, smaller outlet diameter d_{O2}. Thus, the fluid inlet 810 has a larger diameter than the fluid outlet 812 when the device 800 is in the stretched configuration. For example, in certain instances, a ratio of the diameter of the fluid outlet 812 to the fluid inlet 810 may be from about 0.2 to about 0.8, from about 0.3 to about 0.7, or from about 0.4 to about 0.6 while the device 800 is in the stretched configuration. This may restrict fluid flow through the device 800 and redirect fluid away from the heart to the renal arteries and/or other areas of the body, as desired.

In certain instances, the device 800 is adjustable between the relaxed configuration and the stretched configuration so that fluid flow through the device 800 can be adjusted and/or redirected any amount as desired. In certain instances, the device 800 adjusts with changing blood pressure within the patient's body. For example, under increased blood pressure, the adjustable portion 800b lengthens from the relaxed configuration to the stretched configuration and restricts blood flow, causing redirection of blood flow as desired. When blood pressure decreases, the device 800 relaxes back to the relaxed configuration and increases blood flow through the device 800.

In certain instances, the adjustable portion 800b may increase or decrease a blood flow rate, within the vessel (aorta) distal to the side branches (*e.g.,* renal arteries), by between about 5% and about 30% as compared to normal flow. The adjustable portion 700b may occlude the aorta distal to the side branches (*e.g.,* renal arteries) by between about 5% and about 30% to increase blood flow into the kidneys. In certain instances, the adjustable portion 800b is configured to induce stenosis of the aorta of the patient distal of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 800 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 800 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 800 may augment perfusion from a tributary vessel (*e.g.,* renal veins) terminating in the vena cava by altering pressure within the vena cava to alter blood flow from the tributary vessel of the vena cava. In certain instances, the device 800 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

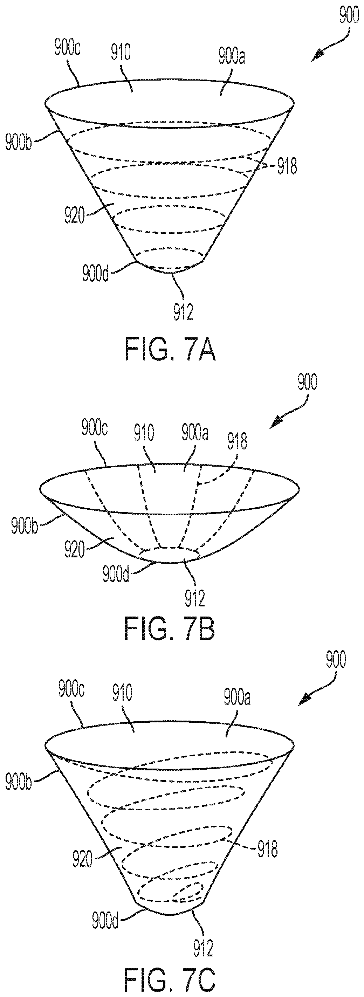

FIGS. 7A-7C show an example implantable medical device 900 that includes a restricting portion 900a and an adjustable portion 900b in accordance with various aspects of the present disclosure. In certain instances, the device 900 may be arranged with a patient's aorta. For example, the device 900 may be arranged with the aorta distal of the renal arteries (not shown).

In certain instances, the device 900 may be, for example, an implantable, expandable stent structure. The device 900 may have multiple layers, including the adjustable portion 900b and the restricting portion 900a, for example. In certain instances, the restricting portion 900a may be, for example, a membrane having central orifices to allow for fluid flow therethrough. For example, the restricting portion 900a may form a channel through the adjustable portion 900b to allow fluid flow therethrough.

In certain instances, the adjustable portion 900b of the device 900 may be substantially conical or tapered in shape. According to the invention, a first end 900c of the device 900 has a larger diameter than a second end 900d of the device 900. As shown, the restricting portion 900a can be located in the center of the adjustable portion 900b. The restricting portion 900a forms a channel through the adjustable portion 900b to allow fluid flow therethrough (e.g., from the first end 900c to the second end 900d).

According to the invention, the device 900 is adjustable between a relaxed configuration and a stretched configuration. As the device 900 lengthens from the relaxed configuration to the stretched configuration, the fluid outlet 912 changes from the first outlet diameter d_{O1} to a second, smaller outlet diameter d_{O2}. Thus, the fluid inlet 910 has a larger diameter than the fluid outlet 912 when the device 900 is in the stretched configuration. For example, in certain instances, a ratio of the diameter of the fluid outlet 912 to the fluid inlet 910 may be from about 0.2 to about 0.8, from about 0.3 to about 0.7, or from about 0.4 to about 0.6 while the device 900 is in the stretched configuration. This may restrict fluid flow through the device 900 and redirect fluid away from the heart to the renal arteries and/or other areas of the body, as desired. In certain instances, the device 900 adjusts between the relaxed configuration and the stretched configuration with changing blood pressure within the patient's body. For example, under increased blood pressure, the adjustable portion 900b lengthens from the relaxed configuration to the stretched configuration and restricts blood flow, causing redirection of blood flow as desired. When blood pressure decreases, the device 900 relaxes back to the relaxed configuration and increases blood flow through the device 900.

FIG. 7A shows an example flow restriction device 900 that includes a restricting portion 900a and an adjustable portion 900b. As shown, the device 900 is conical or tapered in shape so that the first end 900c has a larger diameter than the second end 900d. The device 900 includes a series of stacked ring structures 918, for example Nitinol (NiTi) or stainless steel stent rings. The ring structures 918 may be connected to one another via a membrane material 920. The membrane material 920 may be sufficiently flexible to allow the ring structures 918 to move longitudinally with respect to adjacent ring structures 918 such that the device 900 moves between the relaxed configuration and the stretched configuration.

The ring structures 918 may have varying diameters. For example, a ring structure 918 near the first end 900c of the device 900 may have a larger diameter than a ring structure 918 near the second end 900d of the device 900 such that the device 900 is tapered in shape.

FIGS. 7B and 7C show an example flow restriction device 900 that includes a restricting portion 900a and an adjustable portion 900b. As shown, the device 900 is conical or tapered in shape so that the first end 900c has a larger diameter than the second end 900d. The device 900 includes a spiral ring structure 918, for example a spiral stent wire fabricated of Nitinol (NiTi) or stainless steel. The spiral ring structure 918 is coupled to the membrane material 920. The membrane material 920 may be sufficiently flexible to allow the spiral ring structure 918 to longitudinally expand and contract such that the device 900 moves between the relaxed configuration shown in FIG. 7B and the stretched configuration shown in FIG. 7C. The device 900 may move freely between the relaxed and stretched configurations. For example, the device 900 may move between the relaxed and stretched configurations in response to changes in blood pressure within the patient's body, as discussed herein.

In certain instances, the adjustable portion 900b may increase or decrease a blood flow rate, within the vessel (aorta) distal to the side branches (*e.g.,* renal arteries), by between about 5% and about 30% as compared to normal flow. The adjustable portion 900b may occlude the aorta distal to the side branches (*e.g.,* renal arteries) by between about 5% and about 30% to increase blood flow into the kidneys. In certain instances, the adjustable portion 900b is configured to induce stenosis of the aorta of the patient distal of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 900 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 900 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 900 may augment perfusion from a tributary vessel (*e.g.,* renal veins) terminating in the vena cava by altering pressure within the vena cava to alter blood flow from the tributary vessel of the vena cava. In certain instances, the device 900 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

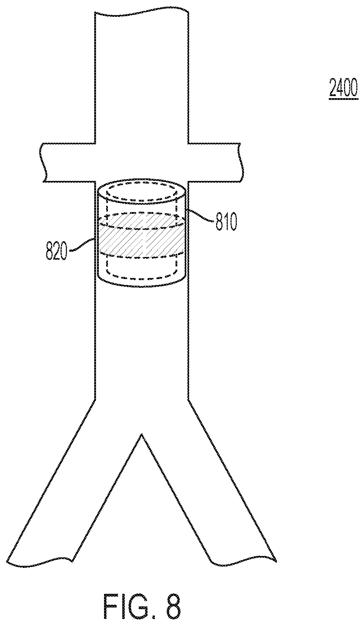

FIG. 8 shows an example implantable medical device 2400 including a body portion 810 and an adjustable portion 820 (not according to claim 1). The body portion 810 is configured to engage the vessel wall and alter blood flow in the vessel of the patient. The adjustable portion 820 is configured to apply a desired amount of restriction to the body portion 810 to alter the blood flow within the vessel, which may in turn increase blood flow into one or more branch vessels extending from the vessel.

When implanted in the aorta, the device 2400 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 2400 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 2400 may augment perfusion from a tributary vessel (*e.g.,* renal veins) terminating in the vena cava by altering pressure within the vena cava to alter blood flow from the tributary vessel of the vena cava. In certain instances, the device 2400 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

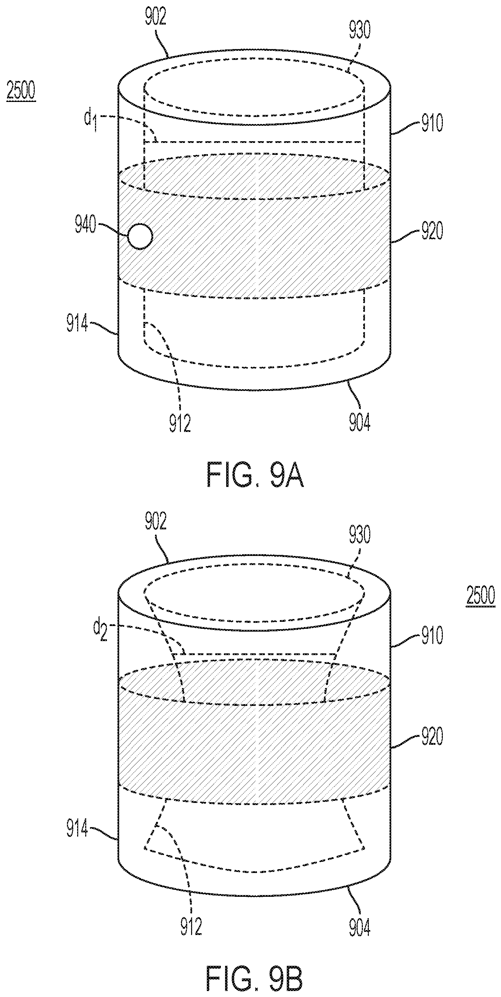

FIG. 9A shows an example implantable medical device 2500 including a body portion 910 and an adjustable portion 920 in a first configuration (not according to claim 1). As shown, the adjustable portion 920 is arranged with the body portion 910. For example, in some instances, the body portion 910 includes an inner wall 912 and an outer wall 914 and the adjustable portion 920 is located between the inner and outer walls 912, 914. The inner wall 912 of the body portion 910 forms a lumen 930 extending from a first end 902 of the device 2500 to a second end 904 of the device for fluid flow therethrough. As shown, the lumen 930 has a first diameter d₁ when the device 2500 is in the first configuration.

In some instances, the adjustable portion 920 may form a chamber between the inner wall 912 and outer wall 914 capable of expanding when fluid is introduced therein. The adjustable portion 920 can include an elastomeric material configured to expand when exposed to fluid. For example, fluid may be injected or otherwise introduced into the adjustable portion 920 to expand the adjustable portion 920 from the first configuration to a second configuration.

FIG. 9B shows the example implantable medical device 2500, shown in FIG. 9A, in the second configuration. As shown, introducing fluid into the adjustable portion 920 causes the lumen 930 to constrict to a smaller diameter (e.g., from the first diameter d₁ to a smaller second diameter d₂). The adjustable portion 920 is configured to expand to decrease the first diameter d₁ of the lumen 930 and contract to increase the second diameter d₂ of the lumen 930 as desired.

In some instances, the device 2500 includes a port 940 arranged on the outer wall 914 of the body portion 910 configured to allow access to the adjustable portion 920, for example, for injecting fluid into the adjustable portion 920.

Any amount of fluid can be introduced into the adjustable portion 920 to constrict the lumen 930 to any size diameter to adjust fluid flow through the device 2500 as desired. For example, the adjustable portion 920 can be configured to increase flow into one or more side branches off the vessel by about 10% and about 30% to improve kidney perfusion and diuresis. In another example, the adjustable portion 920 is configured to reduce the diameter d₁ of the lumen 930 to induce stenosis of the vessel distal of the one or more branch vessels between about 50% and about 70%. In certain instances, fluids such as liquids or gasses may be introduced into the adjustable portion 920 to alter dimension of the device 2500. The adjustable portion 920 may also include a super absorbent polymer or polymers that activated in response to fluids (*e.g.,* saline, water) injected into the adjustable portion 920. The super absorbent polymer may cause the adjustable portion 920 to expand to a pre-set or desired dimeter when fluids are injected. Reversal of the expansion may occur with a secondary fluid infusion. The adjustable portion 920 may also include an electroactive polymer configured to expand or contract in response to electrical energy. The amount of expansion and contraction may be controlled based on the pulsatile flow of blood. Further, the adjustable portion 920 may also include a non-Newtonian fluid or fluids configured to contract and relax in response to pulsatile flow.

In certain instances, the adjustable portion 920 may increase or decrease a fluid flow rate of blood flow, within the vessel (aorta) distal to the side branches (*e.g.,* renal arteries), by between about 5% and about 30% as compared to normal flow. The adjustable portion 920 may occlude the aorta distal to the side branches (*e.g.,* renal arteries) by between about 5% and about 30% to increase blood flow into the kidneys. In certain instances, the adjustable portion 920 is configured to induce stenosis of the aorta of the patient at least partially overlapping of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

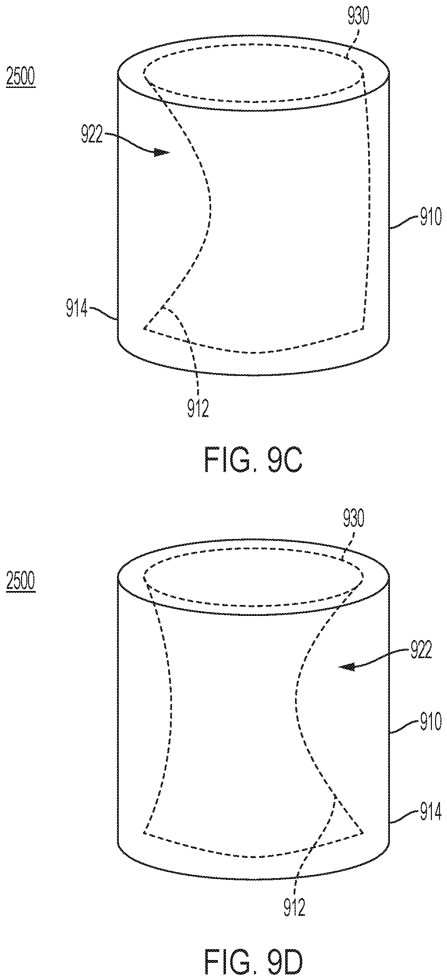

FIG. 9C shows the example implantable medical device 2500, shown in FIGS. 9A-B, in a third configuration. As shown, the adjustable portion 920 may include one or more pockets formed within the chamber between the inner wall 912 and outer wall 914 of the device 2500. The pockets, like the chamber, are capable of expanding when fluid is introduced therein. As shown in FIG. 9C, fluid may be injected into a first pocket 922 to partially occlude and manipulate the shape of the lumen 930. FIG. 9D shows fluid injected into a second pocket 924 to partially occlude and manipulate the shape of the lumen 930. One or both of the first and second pockets 922, 924 may be filled with fluid to adjust the diameter of the device 2500 and fluid flow through the device 2500 as desired.

When implanted in the aorta, the device 2500 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 2500 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 2500 may augment perfusion from a tributary vessel (*e.g.,* renal veins) terminating in the vena cava by altering pressure within the vena cava to alter blood flow from the tributary vessel of the vena cava. In certain instances, the device 2500 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

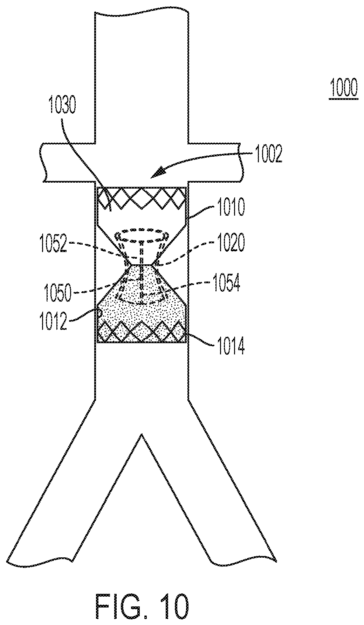

FIG. 10 shows another example implantable medical device 1000 (not according to claim 1). The device 1000 includes a body portion 1010 configured to engage the vessel wall and alter blood flow in the vessel and an adjustable portion 1020 configured to adjust a desired amount of fluid flow through the body portion 1010. As shown, the adjustable portion 1020 is arranged with the body portion 1010. In some instances, the body portion 1010 includes an inner wall 1012 and an outer wall 1014. The inner wall 1012 of the body portion 1010 forms a lumen 1030 extending from a first end 1002 of the device 1000 to a second end 1010 of the device for fluid flow therethrough.

The adjustable portion 1020 includes a flexible element 1050 configured to move between an open position and a closed position. The flexible element 1050 can include a first adjustable element 1052 and a second adjustable element 1054 arranged with the first adjustable element 1052. The first and second adjustable elements 1052, 1054 generally form an outlet within the body portion 1010 for fluid flow therethrough. In some instances, the first and second adjustable elements 1052, 1054 are configured to move between an open position and a closed position. A diameter of the outlet is configured to increase in response to the adjustable elements 1052, 1054 being in the open position and decreased when the adjustable elements 1052, 1054 are in the closed position. The adjustable portion 1020 may be docked within the body portion 1010 and may be replaceable.

In certain instances, the adjustable portion 1020 may increase or decrease a fluid flow rate of blood flow, within the vessel (aorta) distal to the side branches (*e.g.,* renal arteries), by between about 5% and about 30% as compared to normal flow. The adjustable portion 1020 may occlude the aorta distal to the side branches (*e.g.,* renal arteries) by between about 5% and about 30% to increase blood flow into the kidneys. In certain instances, the adjustable portion 1020 is configured to induce stenosis of the aorta of the patient at least partially overlapping of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 1000 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1000 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1000 may augment perfusion from a tributary vessel (*e.g.,* renal veins) terminating in the vena cava by altering pressure within the vena cava to alter blood flow from the tributary vessel of the vena cava. In certain instances, the device 1000 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

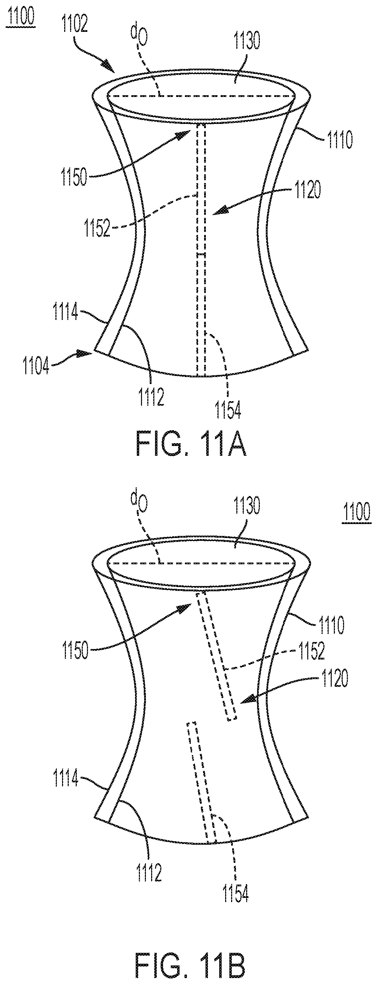

FIG. 11A shows another example implantable medical device 1100 (not according to claim 1). The device 1100 includes a body portion 1110 configured to engage the vessel wall and alter blood flow in the vessel and an adjustable portion 1120 configured to adjust a desired amount of fluid flow through the body portion 1110. As shown, the adjustable portion 1120 is arranged with the body portion 1110. In some instances, the body portion 1110 includes an inner wall 1112 and an outer wall 1114. The inner wall 1112 of the body portion 1110 forms a lumen 1130 extending from a first end 1102 of the device 1100 to a second end 1104 of the device for blood flow therethrough.

The adjustable portion 1120 includes a flexible element 1150 configured to move between a first configuration and a second configuration. The flexible element 1150 can include a first adjustable element 1152 and a second adjustable element 1154 arranged with the first adjustable element 1152. In some instances, the first and second adjustable elements 1152, 1154 are arranged vertically within the body portion 1110, as shown.

The first and second adjustable elements 1152, 1154 generally form an outlet within the body portion 1110 for fluid flow therethrough. In some instances, the first and second adjustable elements 1152, 1154 are configured to move between a first configuration and a second configuration. A diameter dₒ of the outlet is configured to increase in response to the adjustable elements 1152, 1154 being in the first position and decreased when the adjustable elements 1152, 1154 are in the second position.

FIG. 11B shows the implantable medical device, shown in FIG. 11A, in the second configuration in accordance with various aspects of the present disclosure. As shown, the first adjustable element 1152 and the second adjustable element 1154 are separated from one another forming a gap for fluid flow therethrough.

In certain instances, the adjustable portion 1120 may increase or decrease a fluid flow rate of blood flow, within the vessel (aorta) distal to the side branches (*e.g.,* renal arteries), by between about 5% and about 30% as compared to normal flow. The adjustable portion 1120 may occlude the aorta distal to the side branches (*e.g.,* renal arteries) by between about 5% and about 30% to increase blood flow into the kidneys. In certain instances, the adjustable portion 1120 is configured to induce stenosis of the aorta of the patient at least partially overlapping of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 1100 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1100 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1100 may augment perfusion from a tributary vessel (*e.g.,* renal veins) terminating in the vena cava by altering pressure within the vena cava to alter blood flow from the tributary vessel of the vena cava. In certain instances, the device 1100 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

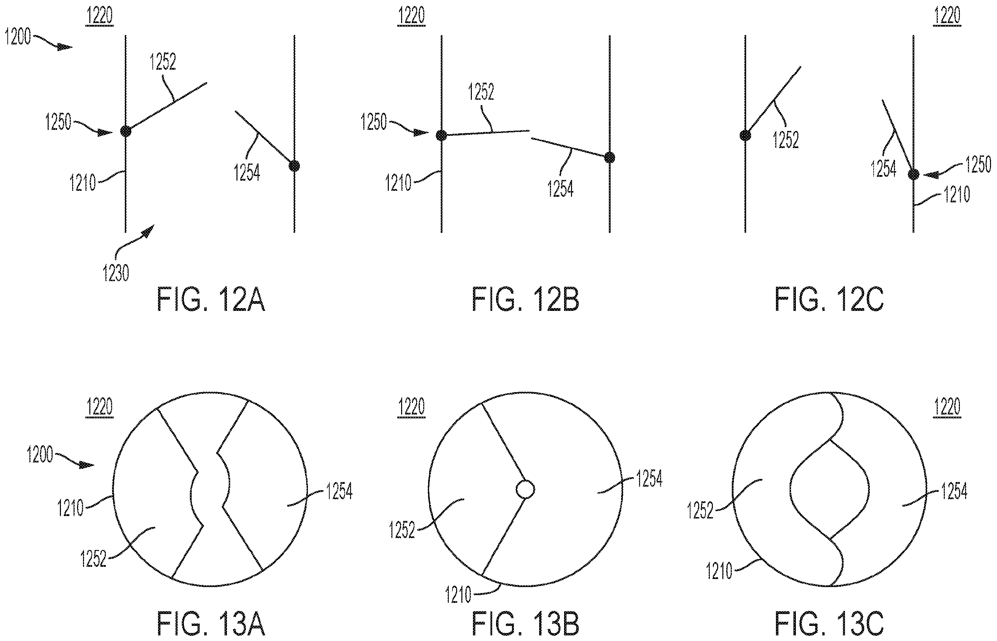

FIG. 12A shows an example adjustable portion 1220 of an implantable medical device 1200 in a first configuration (not according to claim 1). The adjustable portion 1220 is configured to adjust a desired amount of fluid flow through the body portion 1210 of the device 1200. For example, the device 1200 may adjust the fluid flow through the body portion 1210 a certain amount in response to blood pressure. As shown, the adjustable portion 1220 is arranged with the body portion 1210. In some instances, the body portion 1210 includes an inner wall 1212 and an outer wall 1214. The inner wall 1212 of the body portion 1210 forms a lumen 1230 extending from a first end 1202 of the device 1200 to a second end 1204 of the device for blood flow therethrough.

The adjustable portion 1220 includes a flexible element 1250 configured to move between a first configuration, a second configuration, and a third configuration. The flexible element 1250 can also be a rigid or semi-rigid element. FIG. 12A shows a first adjustable element 1252 and a second adjustable element 1254 arranged with the first adjustable element 1252. In some instances, the first and second adjustable elements 1252, 1254 are arranged horizontally within the body portion 1210, as shown. In other terms, the first and second adjustable elements 1252, 1254 may be attached to the inner wall 1212 of the body portion 1210. In various embodiments, the first and second adjustable elements 1252, 1254 may be arranged vertically within the body portion 1210 or in any other suitable configuration capable of adjusting fluid flow as desired.

The first and second adjustable elements 1252, 1254 generally form an outlet within the body portion 1210 for fluid flow therethrough. In some instances, the first and second adjustable elements 1252, 1254 are configured to move between the first configuration and the second configuration. A diameter dₒ of the outlet is configured to increase in response to the adjustable elements 1252, 1254 being in the first position and decreased when the adjustable elements 1252, 1254 are in the second position.

FIG. 12B shows the adjustable portion 1220, shown in FIG. 12A, in the second configuration. As shown, the second configuration decreases the diameter dₒ of the outlet or, in some instances, may be configured to completely occlude the outlet altogether.

FIG. 12C shows the adjustable portion 1220, shown in FIGs. 12A-B, in the third configuration. As shown, the adjustable elements 1252, 1254 are movable between the second configuration and the third configuration such that fluid flow through the lumen 1230 may be increased. It should be known that the first and second adjustable elements 1252, 1254 can be positioned in any configuration to increase or decrease fluid flow through the body portion 1210 of the device 1200 as desired.

In certain instances, the adjustable portion 1220 may increase or decrease a fluid flow rate within the vessel (aorta) distal to the side branches (*e.g.,* renal arteries), by between about 5% and about 30% as compared to normal flow. The adjustable portion 1220 may occlude the aorta distal to the side branches (*e.g.,* renal arteries) by between about 5% and about 30% to increase blood flow into the kidneys. In certain instances, the adjustable portion 1220 is configured to induce stenosis of the aorta of the patient distal of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 1200 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1200 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1200 may augment perfusion from a tributary vessel (*e.g.,* renal veins) terminating in the vena cava by altering pressure within the vena cava to alter blood flow from the tributary vessel of the vena cava. In certain instances, the device 1200 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

FIG. 13A-C shows a top view of the example adjustable portion shown in FIGS. 12A-12C in a first configuration (not according to claim 1). The adjustable portion 1220 is configured to adjust a desired amount of fluid flow through a body portion 1210 (not shown) of the device 1200. The adjustable portion 1220 is arranged with the body portion 1210 of the device 1200.

The adjustable portion 1220 includes a first adjustable element 1252 and a second adjustable element 1254 arranged with the first adjustable element 1252. As discussed with reference to FIG. 12A, the adjustable elements can be rigid, semi-rigid, or flexible elements. In some instances, the first adjustable element 1252 is an upper flap and the second adjustable element 1254 is a lower flap arranged beneath the upper flap. At least one of the upper flap and the lower flap is configured to hinge to adjust the amount of fluid flow through the body portion 1210 of the device 1200.

The first and second adjustable elements 1252, 1254 generally form an outlet within the body portion 1210 for fluid flow therethrough. In some instances, the first and second adjustable elements 1252, 1254 are configured to move between the first configuration, a second configuration, and a third configuration. A diameter dₒ of the outlet is configured to increase in response to the adjustable elements 1252, 1254 being in the first configuration and decreased when the adjustable elements 1252, 1254 are in the second configuration.

FIG. 13B shows the adjustable portion 1220, shown in FIG. 13A, in the second configuration. As shown, as the upper and lower flaps hinge closed, they overlap one another forming the second configuration. The second configuration decreases the diameter dₒ of the outlet.

FIG. 13C shows the adjustable portion 1220, shown in FIGs. 13A-B, in the third configuration. As shown, the adjustable elements 1252, 1254 are movable between the second configuration and the third configuration such that fluid flow through the body portion 1210 may be increased. It should be known that the first and second adjustable elements 1252, 1254 can be positioned in any configuration to increase or decrease fluid flow through the body portion 1210 of the device 1200 as desired. In certain instances, the adjustable portion 1220 alters a level of restriction to blood flow based on pressure. As pressure increases, the adjustable portion 1220 may open and allow more flow and close in rest.

In certain instances, the adjustable portion 1220 may increase or decrease a blood flow rate, within the vessel (aorta) distal to the side branches (*e.g.,* renal arteries), by between about 5% and about 30% as compared to normal flow. The adjustable portion 1220 may occlude the aorta distal to the side branches (*e.g.,* renal arteries) by between about 5% and about 30% to increase blood flow into the kidneys. In certain instances, the adjustable portion 1220 is configured to induce stenosis of the aorta of the patient at least partially overlapping of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

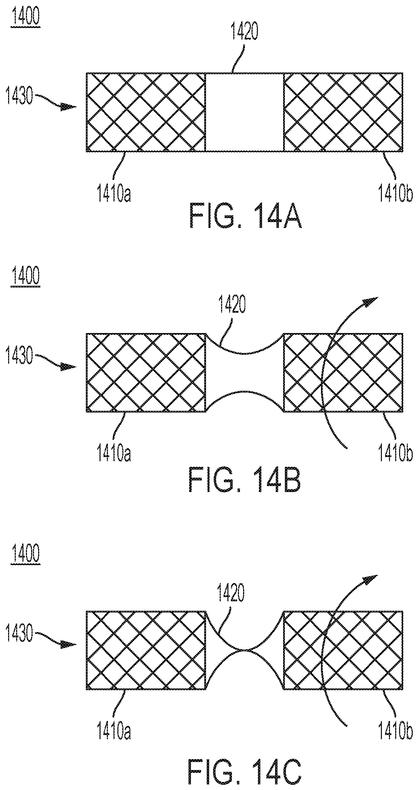

FIG. 14A-C shows an example implantable medical device 1400 including a first body portion 1410a configured to engage the vessel wall, a second body portion 1410b configured to engage the vessel wall, and an adjustable portion 1420 in various configurations (not according to claim 1). The adjustable portion 1420 is arranged between the first body portion 1410a and the second body portion 1410b and forms a lumen 1430 therethrough. In some instances, the first and second body portions 1410a, 1410b include stent elements and the adjustable portion 1420 includes a membrane. The adjustable portion 1420 is generally configured to rotate to alter an amount of fluid flow through the lumen 1430.

FIG. 14A shows the device 1400 in a first, open configuration allowing fluid flow through the first and second body portions 1410a, 1410b of the device 1400. As shown, in the first configuration, the adjustable portion 1420 is substantially straight (e.g., the adjustable portion 1420 is not rotated) to allow fluid flow through the lumen 1430.

FIG. 14B shows the device 1400 in a second, partially constricted configuration. In some instances, the first body portion 1410a may be fixed within the patient's body so that the first body portion 1410a is not movable within the body. The second body portion 1410b may be free such that the second body portion 1410b can rotate relative to the first body portion 1410a and constrict the adjustable portion 1420. For example, the second body portion 1410b can be twisted approximately 90 degrees in either a clockwise or counterclockwise direction to reduce a diameter of the adjustable portion 1420.

FIG. 14C shows the device 1400 in a third, constricted configuration. For example, the second body portion 1410b can be twisted approximately 1140 degrees in either a clockwise or counterclockwise direction to reduce the diameter of the adjustable portion 1420.

In certain instances, the adjustable portion 1420 may increase or decrease a fluid flow rate of blood flow, within the vessel (aorta) distal to the side branches (*e.g.,* renal arteries), by about 10% and about 30% as compared to normal flow. The adjustable portion 1420 may occlude the aorta distal to the side branches (e.g., renal arteries) by about 10% and about 30% to increase blood flow into the kidneys. In certain instances, the adjustable portion 1420 is configured to induce stenosis of the aorta of the patient at least partially distal of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 1400 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1400 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1400 may augment perfusion from a tributary vessel (*e.g.,* renal veins) terminating in the vena cava by altering pressure within the vena cava to alter blood flow from the tributary vessel of the vena cava. In certain instances, the device 1400 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

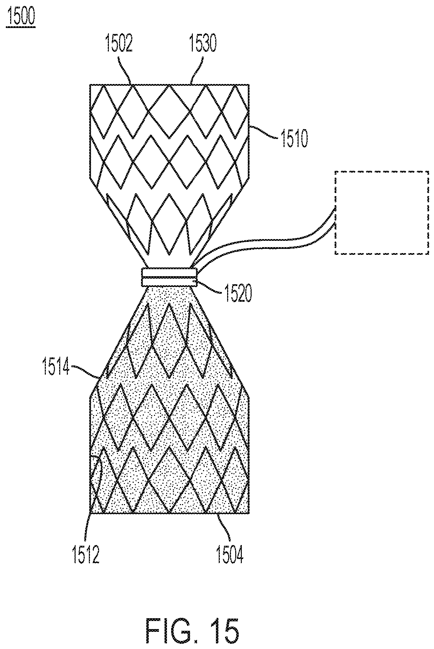

FIG. 15 shows an example implantable medical device 1500 including a body portion 1510 and an adjustable portion 1520 about an external surface 1514 of the body portion (not according to claim 1). The body portion 1510 is configured to engage the vessel wall and alter blood flow in the vessel and the adjustable portion 1520 is configured to adjust a desired amount of fluid flow through the body portion 1510. As shown, the adjustable portion 1520 is arranged with the body portion 1510. In some instances, the body portion 1510 includes an inner surface 1512 and an outer surface 1514 and the adjustable portion 1520 is arranged around the outer surface 1514 of the body portion 1510. The inner surface 1512 of the body portion 1510 forms a lumen 1530 extending from a first end 1502 of the device 1500 to a second end 1504 of the device 1500 for fluid flow therethrough.

As shown, the implantable flow restriction device 1500 may be a flow restricting stent graft. The flow restricting stent graft device 1500 may be diametrically adjustable. The flow restricting stent graft device 1500 may be diametrically adjusted multiple times to achieve a desired shape and diameter(s). In certain instances, the flow restricting stent graft device 1500 may be diametrically adjustable by way of a constricting force applied to the external surface 1514 of the flow restricting stent graft device 1500. The constricting force may be applied by way of the adjustable portion 1520, for example. The flow restricting stent graft device 1500 may include a stent and a graft structure. Either or both of the stent and the graft structure of the flow restricting stent graft device 1500 may be configured to reduce in diameter in response to the constricting force imparted by the adjustable portion 1520.

In some instances, the adjustable portion 1520 is a cinching member configured to constrict the body portion 1510 to reduce the diameter of the body portion 1510. The cinching member may be adjusted or tensioned in a variety of ways. For example, the cinching member may be tensioned by using electroactive polymers and/or Nitinol (NiTi) in coil configuration to tighten and restrict to the adjustable portion 1520.

In some instances, the adjustable portion 1520 includes a heat inductive material configured to reduce the diameter of the body portion 1510 in response to heat applied to the heat inductive material.

In some instances, the adjustable portion 1520 may be coupled to an adjusting device 1522 configured to adjust the tightening and restricting of the adjustable portion 1520. The adjusting device 1522 may include, for example, circuitry, sensing capability, and/or flow algorithms configured to execute commands that adjust tension of the adjustable portion 1520. The adjusting device 1522 may adjust based on a user (e.g., a physician) or one or more physiological parameters of the patient. For example, the medical device 1522 may prompt altering of the adjustable portion 1520 when a patient exercises or rests. The medical device 1522 may prompt altering of the adjustable portion 1520 when a patient is laying down or standing. In addition, the medical device 1522 may prompt altering of the adjustable portion 1520 in response to back pressure.

In certain instances, the adjustable portion 1520 may increase or decrease a fluid flow rate of blood flow, within the vessel (aorta) distal to the side branches (*e.g.,* renal arteries), by about 10% and about 30% as compared to normal flow. The adjustable portion 1520 may occlude the aorta distal to the side branches (*e.g.,* renal arteries) by about 10% and about 30% to increase blood flow into the kidneys. In certain instances, the adjustable portion 1520 is configured to induce stenosis of the aorta of the patient at least partially distal of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 1500 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1500 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1500 may augment perfusion from a tributary vessel (*e.g.,* renal veins) terminating in the vena cava by altering pressure within the vena cava to alter blood flow from the tributary vessel of the vena cava. In certain instances, the device 1500 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

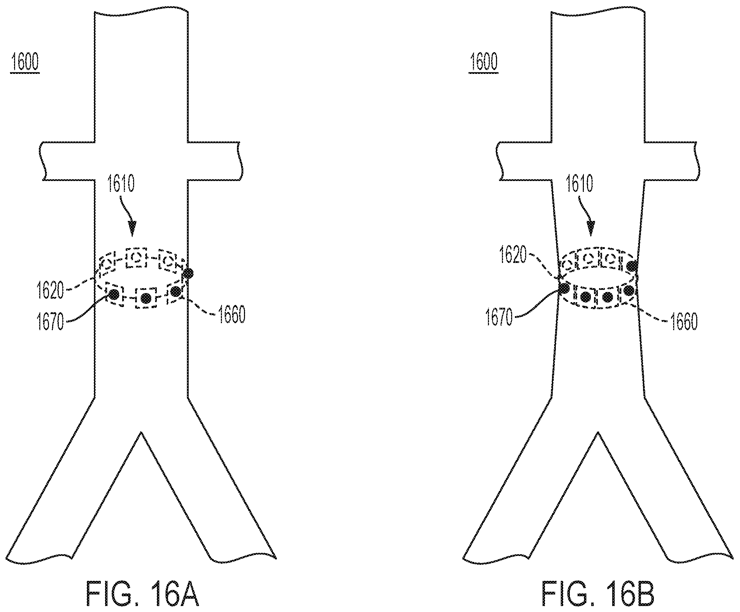

FIG. 16A-B shows an example implantable medical device 1600 including an adjustable portion 1610 in various configurations (not according to claim 1). The adjustable portion 1610 is configured to engage the vessel wall and alter blood flow in the vessel. For example, the adjustable portion 1610 may include a plurality of anchors 1670 configured to anchor the device 1600 within the vessel. In some instances, the adjustable portion 1610 includes an adjustable member 1620 configured to adjust a desired amount of fluid flow through the adjustable portion 1610. In some instances, the adjustable portion 1610 includes a plurality of segments 1660 arranged circumferentially within the vessel. The adjustable member 1620 may be, for example, a cinching member configured to bring the plurality of segments 1660 closer together to decrease the diameter of the adjustable portion 1610 of the device 1600.

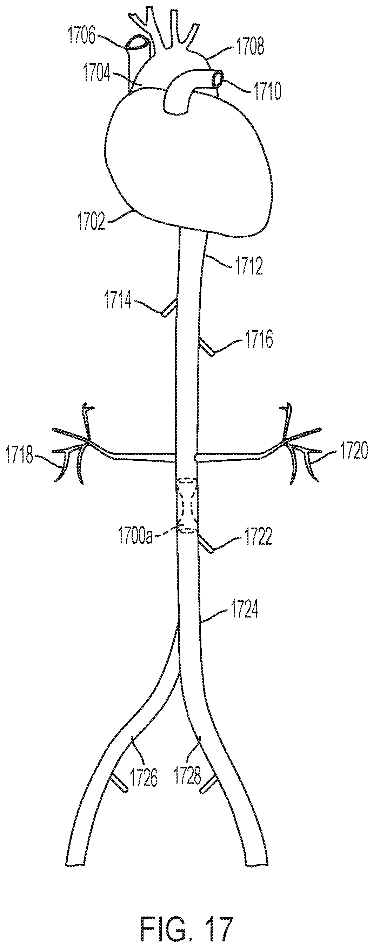

FIG. 17 shows an example implantable medical device 1700a and an expandable device 1700b (not according to claim 1). In certain instances, the adjustable portion 1010 may increase or decrease a fluid flow rate of blood flow, within the vessel (aorta) distal to the side branches (*e.g.,* renal arteries), by about 10% and about 30% as compared to normal flow. The adjustable portion 1010 may occlude the aorta distal to the side branches (*e.g.,* renal arteries) by about 10% and about 30% to increase blood flow into the kidneys. In certain instances, the adjustable portion 1010 is configured to induce stenosis of the aorta of the patient at least partially distal of the side branches (e.g., renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

When implanted in the aorta, the device 1600 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1600 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1600 may augment perfusion from a tributary vessel (*e.g.,* renal veins) terminating in the vena cava by altering pressure within the vena cava to alter blood flow from the tributary vessel of the vena cava. In certain instances, the device 1600 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

FIG. 17 shows an example implantable medical device 1700a and an expandable device (not according to claim 1). The implantable flow restriction device 1700a is shown arranged with a patient's aorta. The patient's vasculature shown in FIG. 17 includes the patient's heart 1702, aortic root 1704, superior vena cava 1706, aortic arch 1708, pulmonary trunk 1710, descending aorta 1712, celiac artery 1714, superior mesenteric artery 1716, renal arteries 1718, 1720, inferior mesenteric artery 1722, abdominal aorta 1724, and iliac arteries 1726, 1728. The implantable flow restriction device 100a may arranged with the aorta distal of the renal arteries 1718, 1720.

The implantable flow restriction device 1700 may be configured to increase blood flow into at least one of the renal arteries 1718, 1720. As shown in FIG. 17, the implantable flow restriction device 1700 may be a flow restricting stent graft. The flow restricting stent graft device 1700 may be configured to reduce a diameter of the aorta and/or the iliac arteries 1726, 1728 to increase resistance and reduce blood flow distally thereof. Although FIG. 17 shows the flow restricting stent graft device 1700a arranged within the aorta, a patient may additionally or alternatively include the flow restricting stent graft device 1700a arranged within one or both of the iliac arteries 1726, 1728. The flow restricting stent graft device 1700a may be diametrically adjustable. The restricting stent graft device 1700a may be diametrically adjusted multiple times to achieve a desired shape and diameter(s). In certain instances, the flow restricting stent graft device 1700a may be diametrically adjustable by way of a distensible force applied within the flow restricting stent graft device 1700a. The distensible force may be applied by way of a balloon structure. For example, the balloon structure may vary depending on the desired amount of distensible force and desired degree of stenosis. The flow restricting stent graft device 1700a may include a stent and a graft structure. Either or both of the stent and the graft structure of the flow restricting stent graft device 1700a may be configured to maintain a new diameter in response to the distensible force.

In certain instances, the flow restricting stent graft device 1700a may include stent components that are expandable, for example, by balloon. The flow restricting stent graft device 1700a may include expandable portions or the entire flow restricting stent graft device 1700a may be balloon expandable. The stent components may be formed of stainless steel or another balloon adjustable material. For example, expandable portions of the flow restricting stent graft device 1700a may be configured to have controlled expansion that allows diametric adjustment beyond an initial deployment diameter through a plurality of adjusted diameters up to a maxim diametric expansion limit of the balloon expandable flow restricting stent graft device 1700a. Examples of diametrically adjustable devices and associated methods are also described in U.S. Patent Publication 2016/0143759 to Bohn et al.

In certain instances, the flow restricting stent graft device 1700a may be expanded by an expandable medical device arranged within the flow restricting stent graft device 1700a to alter or adjust physical dimensions of the flow restricting stent graft device 1700a to increase blood flow into at least one branch vessel of the vessel. To set the flow restricting stent graft device 1700a at the altered dimensions, the temperature of the expandable device (*e.g.,* a balloon) is altered to a heat set temperature of the stent elements of the flow restricting stent graft device 1700a to maintain the flow restricting stent graft device 1700a at the adjusted physical dimensions.

When implanted in the aorta, the device 1700 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1700 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1700 may augment perfusion from a tributary vessel (*e.g.,* renal veins) terminating in the vena cava by altering pressure within the vena cava to alter blood flow from the tributary vessel of the vena cava. In certain instances, the device 1700 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

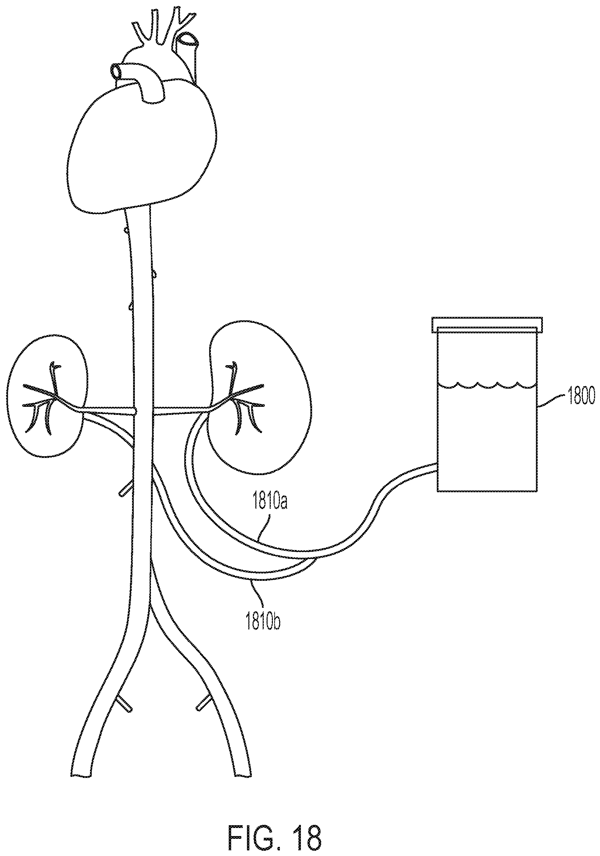

FIG. 18 shows an example vacuum 1800 for adjusting blood flow (not according to claim 1). The vacuum 1800 includes one or more tubes 1810a 1810b configured to implant within one or more branch vessels extending from the vessel. The vacuum 1800 is configured to apply a vacuum force through the one or more tubes to pull blood into the branch vessel.

In some instances, the one or more tubes 1810a, 1810b are configured to implant within one or more kidney veins, and the vacuum is configured to pull blood into one or both kidneys of the patient.

In certain instances, the vacuum 1800 may increase or decrease a fluid flow rate of blood flow, within the vessel (aorta) distal to the side branches (e.g., renal arteries), by between about 5% and about 30% as compared to normal flow. In certain instances, the vacuum 1800 is configured to induce stenosis of the aorta of the patient at least partially overlapping of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches (*e.g.,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

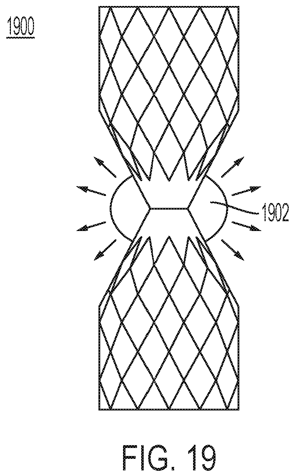

FIG. 19 shows an example implantable medical device 1900 (not according to claim 1). The implantable medical device 1900 may include an adjustable portion 1902. The adjustable portion 1902 may be configured to adjust a desired amount of fluid flow through the adjustable portion 1902. In certain instances, the adjustable portion 1902 may include a bladder configured to expand and contract with pulsatile flow of the heart. The bladder uses the Windkessel effect of the vessel to regulate flow.

In certain instances, the adjustable portion 1902 may increase or decrease a fluid flow rate of blood flow, within the vessel (aorta) distal to the side branches (*e.g.,* renal arteries), by approximately about 10% - about 20%, about 20%-about 30%, about 30% - about 40% or any number therebetween as compared to normal flow. The adjustable portion 1902 may occlude the aorta distal to the side branches (*e.g.,* renal arteries) by approximately about 10% - about 20%, about 20%-about 30%, about 30% - about 40% or any number therebetween to increase blood flow into the kidneys. In certain instances, the adjustable portion 1902 is configured to induce stenosis of the aorta of the patient at least partially distal of the side branches (*e.g.,* renal arteries) between about 40% and about 80% and alter blood flow into one or more of the side branches (*e.g.,* renal arteries) while maintaining a substantially unrestricted blood flow within the vessel (aorta) proximal to one or more of the side branches *(e.g*.*,* renal arteries). In certain instances, the induced stenosis is between about 50% and about 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

A method of applying flow modification therapy generally includes arranging an implantable device with a vessel of a patient, arranging an expandable device (e.g., a balloon) within the implantable medical device and expanding the expandable device within the implantable medical device to adjust physical dimensions of the implantable medical device to increase blood flow into at least one branch vessel of the vessel. The method also includes altering a temperature of the expandable device to a heat set temperature of the implantable medical device to maintain the device at the adjusted physical dimensions. For example, in some instances, the device may include a shape memory material such as Nitinol (NiTi) having an austenite finish (Af) temperature above average body temperature (e.g., about 42 to 45 degrees C) and set to a diameter of about 8 mm. At temperatures below body temperature, the device may behave like a balloon expandable stent capable of being delivered to the vessel in a delivery configuration and expanded to a desired restriction profile to induce improved perfusion to the renal arteries. As the device warms to body temperature, the device may return to its delivery configuration. The device may then be sized up again as needed. In some instances, a warm balloon (e.g., an angioplasty balloon filled with 45 degree C saline solution, for example) may also be inserted into the device to decrease the size of the device as desired.

In some instances, the implantable medical devices can be used to create stenosis and cause an arteriovenous (A-V) fistula to mature and/or close. For example, the implantable medical devices could be used to cycle an A-V graft from a larger diameter (e.g., 6mm) while on dialysis to a smaller diameter (e.g., 4 mm) when not on dialysis. The implantable medical devices can also be used, for example, during Fontan and Hemi-Fontan procedures or during transjugular intrahepatic portosystemic shunt (TIPS) procedure in which the implantable medical devices may redirect blood away from the heart or other areas of the body as desired.

When implanted in the aorta, the device 1900 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 1900 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between about 50% and about 70% of a nominal flow.

When implanted in the vena cava, the device 1900 may augment perfusion from a tributary vessel (*e.g.,* renal veins) terminating in the vena cava by altering pressure within the vena cava to alter blood flow from the tributary vessel of the vena cava. In certain instances, the device 1900 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

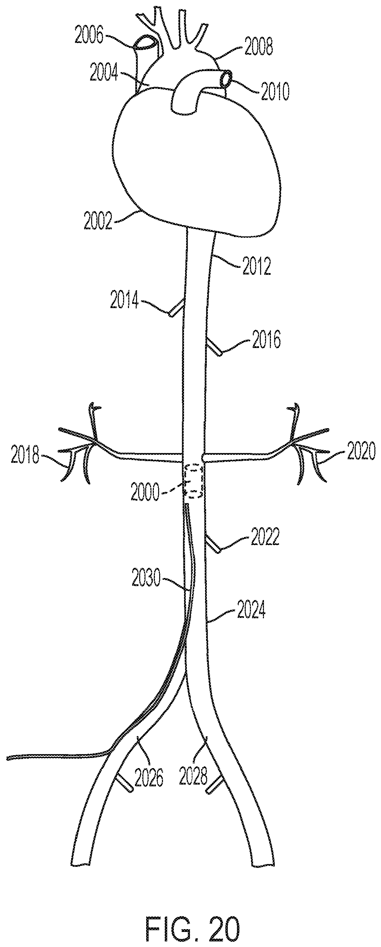

FIG. 20 shows an example implantable restriction device 2000 (not according to claim 1). In certain instances, the implantable restriction device 2000 includes a flow restricting balloon in accordance with various aspects of the present disclosure. The implantable restriction device 2000 may be coupled to a catheter 2030. The evaluation implantable evaluation flow restriction device 2000 is shown arranged with a patient's aorta. The patient's vasculature shown in FIG. 20 includes the patient's heart 2002, aortic root 2004, superior vena cava 2006, aortic arch 2008, pulmonary trunk 2010, descending aorta 2012, celiac artery 2014, superior mesenteric artery 2016, renal arteries 2018, 2020, inferior mesenteric artery 2022, abdominal aorta 2024, and iliac arteries 2026, 2028. The evaluation implantable evaluation flow restriction device 2000 may be arranged with the aorta distal of the renal arteries 2018, 2020.

In certain instances, the evaluation flow restriction device 2000 may be for augmenting perfusion of a branch vessel (*e.g.,* renal arteries 2018, 2020 or iliac arteries 2026, 2028) originating from the aorta permanently or for assessing patient's tolerance to fluid flow diversion/management. The evaluation flow restriction device 2000 may be adjusted by increasing resistance to blood flow through the evaluation flow restriction device 2000 to increase pressure within the aorta to increase blood flow into the branch vessel. For example, the evaluation flow restriction device 2000 may be adjusted by increasing pressure in the flow restricting balloon. For example, as pressure is increased and the flow restricting balloon expands, the diameter of the evaluation flow restriction device 2000 is decreased, thereby restricting flow through the evaluation flow restriction device 2000. As the pressure is decreased and the flow restricting balloon compresses, the diameter of the evaluation flow restriction device 2000 is increased and flow through the evaluation flow restriction device 2000 increases.

Flow modification through the vessel can be evaluated by assessing the patient's tolerance to decreased blood flow distal of at least one branch vessel of the aorta. For example, the evaluation flow restriction device 2000 may be arranged within the aorta and/or other vessel of the patient as desired. Physical dimensions of the evaluation flow restriction device 2000 (i.e., the diameter of the device 2000) may then be adjusted to increase fluid flow into at least one of the branch vessels. For example, the flow restricting balloon may be expanded or contracted.

The patient's tolerance to decreased blood flow may be measured by any of a variety of ways. In some instances, assessing the patient's tolerance to increased blood flow may include measuring one or more of the patient's diuresis rate, distal fractional flow reserve, and hemodynamic response. The assessment may also include measuring the patient's ankle pressure, Doppler ultrasound velocity, and hemodynamic parameters in lower limbs of the patient. In some instances, assessing the patient's tolerance to the decreased blood includes, for example, determining an amount of flow resistance within the vessel for the patient to optimize flow modification without adversely affecting distal perfusion of the vessel and heart load.

In some instances, the dimensions of the vessel may be measured for implant size for an implantable medical device. The evaluation flow restriction device 2000 may be arranged with a vessel of a patient and the physical dimensions of the evaluation flow restriction device 2000 may be adjusted to determine a size of the vessel. In some instances, the patient's tolerance to the decreased blood flow distal to the branch vessel may also be measured as described herein, including measuring the diuresis rate, distal fractional flow reserve, and/or hemodynamic response of the patient.

Once dimensions of the vessel are measured and/or flow tolerance is assessed, an implantable medical device customized or adjustable to the measured dimensions of the vessel may be selected. The evaluation flow restriction device 2000 may be removed after assessing the patient's tolerance. The implantable medical device is then placed within the vessel, the implantable medical device being configured to increase blood flow into one or more branch vessels as described in detail above with reference to FIG. 1. In addition and when implanted in the aorta, the implantable medical device may be configured to increase blood flow into one or more branch vessels such as the renal arteries while maintaining a substantially unrestricted blood flow within the aorta proximal to the one or more branch vessels to influence renal artery pressure and regulate systemic blood pressure. The device 2000 may also be arranged within a vena cava as described in detail herein.

In some instances, the evaluation flow restriction device 2000 may be used as an implantable medical device providing therapy. In these instances, the evaluation flow restriction device 2000 may be configured to optimize flow modification without adversely affecting distal perfusion of the vessel and heart load. For example, the physical dimensions of the evaluation flow restriction device 2000 may be adjusted to optimize flow modification to induce stenosis of the vessel distal of the branch vessel between about 40% and about 80%. In some instances, adjusting the physical dimensions of the evaluation flow restriction device 2000 can also increase urine production, lower serum creatinine, and/or lower plasma NGAL.

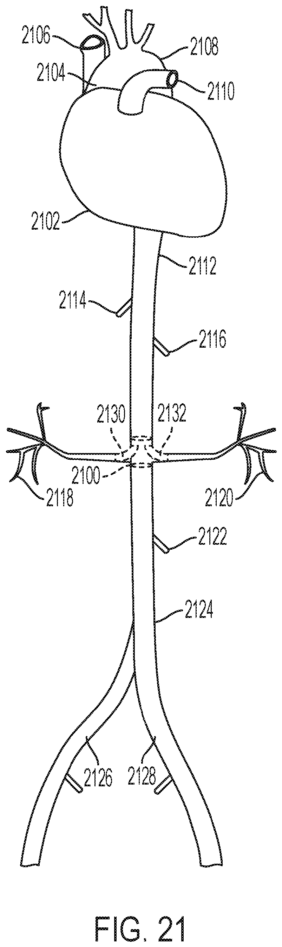

FIG. 21 shows an example implantable medical device 2100 having branches (not according to claim 1). FIG. 21 shows an example flow restriction device that includes an implantable medical device 2100 in accordance with various aspects of the present disclosure. The implantable medical device 2100 is shown arranged with a patient's aorta. The patient's vasculature shown in FIG. 21 includes the patient's heart 2102, aortic root 2104, superior vena cava 2106, aortic arch 2108, pulmonary trunk 2110, descending aorta 2112, celiac artery 2114, superior mesenteric artery 2116, renal arteries 2118, 2120, inferior mesenteric artery 2122, abdominal aorta 2124, and iliac arteries 2126, 2128. The implantable medical device 2100 may be arranged with the aorta distal of the renal arteries 2118, 2120.

The implantable medical device 2100 may be configured to increase blood flow into at least one of the renal arteries 2118, 2120. As shown in FIG. 21, the implantable medical device 2100 may be a flow diversion stent graft device. The implantable medical device 2100 may include one or more branches 2130, 2132. The branches 2130, 2132 may be arranged within the renal arteries 2118, 2120, with a main body portion of the implantable medical device 2100 being arranged within the aorta. The branches 2130, 2132 of the implantable medical device 2100 may direct blood flow into the renal arteries 2118, 2120 to increase blood flow therein. The implantable medical device 2100 allows for blood flow distally to the implantable medical device 2100. The branches 2130, 2132 of the implantable medical device 2100 may increase resistance for blood flow distally of the implantable medical device 2100 thereby increasing blood into the renal arteries 2118, 2120.

The implantable medical device 2100 may also maintain a substantially unrestricted blood flow within the aorta proximal to the renal arteries 2118, 2120. Blood flow may be monitored proximal to the renal arteries 2118, 2120 and the amount of restriction applied via the implantable medical device 2100 may be adjusted to maintain a substantially normal flow to areas of the aorta proximal to the renal arteries 2118, 2120 and also to increase blood flow into at least one of the renal arteries 2118, 2120.

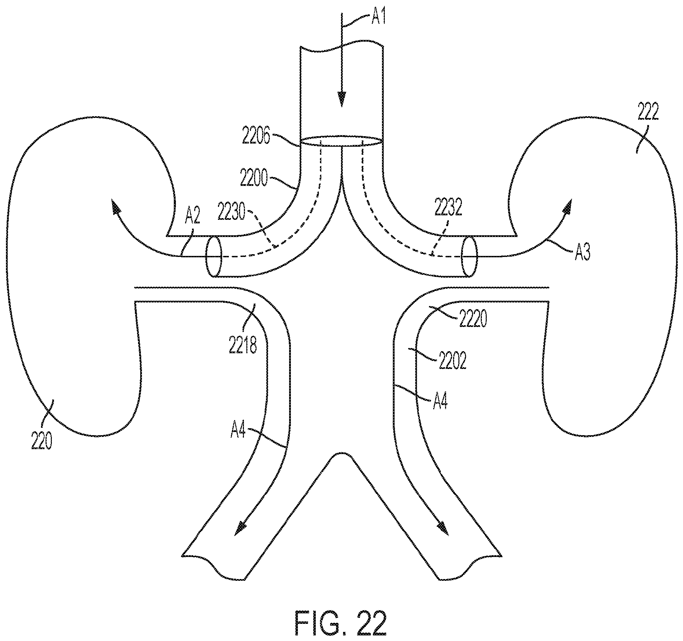

FIG. 22 shows another example implantable medical device 2200 having branches arranged within a vessel 2202 and side branch vessels 2218, 2220 in accordance with various aspects of the present disclosure. The vessel 2202 may be the aorta and the branch vessels 2218, 2220 may be the renal arteries. As shown in FIG. 22, the implantable medical device 2200 is arranged with a patient's aorta and the branch vessels 2218, 2220 are the renal arteries.

The implantable medical device 2200 may be configured to increase blood flow into at least one of the kidneys 2240, 2242. As shown in FIG. 22, the implantable medical device 2200 may be a flow diversion stent graft device. The implantable medical device 2200 may include one or more branches 2230, 2232. The branches 2230, 2232 may be arranged within the branch vessels (renal arteries) 2218, 2220, with a main body portion 2206 of the implantable medical device 2200 being arranged within the vessel (aorta) 2202. The branches 2230, 2232 of the implantable medical device 2200 may direct blood flow into the kidneys 2240, 2242 to increase blood flow therein when the device is arranged within the aorta and branch vessels such as the renal arteries (or celiac, hepatic, or mesenteric arteries).

As shown in FIG. 22, the branches 2230, 2232 are arranged within the branch vessels 2218, 2220 to allow retrograde flow from the branch vessels 2218, 2220 back into the vessel 2202. In instances where the implantable medical device 2200 is arranged within the aorta and the branches 2230, 2232 are placed within the renal arteries, the branches 2230, 2232 are configured to allow retrograde flow from the renal arteries into the aorta). The branches 2230, 2232 of the implantable medical device 2200 can be sized to have a smaller diameter than the branch vessels 2218, 2220 to allow retrograde flow. In addition, the branches 2230, 2232 may be placed at a length within the branch vessels 2218, 2220 to allow retrograde flow. The branches 2230, 2232 may be sized in length, such that flow is delivered directly into the kidneys, or such that the flow is delivered to a location within the renal arteries, allowing desired outcomes such as pressure and/or flow to the kidneys.

In certain instances, the main body portion 2206 of the implantable medical device 2200 may contact the vessel walls of the vessel (aorta) 2202. As a result, the main body portion 2206 of the implantable medical device 2200 may block blood from flowing through the implantable medical device 2200 within the vessel 2202 distal to the implantable medical device 2200. The branches 2230, 2232 being configured to allow retrograde flow maintains blood flow within the vessel 2202. In addition, the branches 2230, 2232 being configured to allow retrograde flow and the main body portion 2206, being configured to block blood from flowing directly through the implantable medical device 2200 distally, can increase resistance to blood flow within the vessel 2202 and further increase blood flow into the branch vessels 2218, 2220.

The direction of fluid flow through the implantable medical device 2200 is shown by arrows A1-A4. For example, fluid flows into the main body portion of the device, as shown with arrow A1. Fluid is then diverted into either of the branches 2230, 2232 of the implantable medical device 2200 and redirected toward the kidneys 2240, 2242 as shown by arrows A2 and A3. Fluid then flows out of the kidneys 2240, 2242 (or other organs when the implantable medical device 2200 is implanted into a different vessel) as shown by arrows A4 and A4.

In certain instances, the implantable medical device 2200 may increase or decrease the fluid flow rate within the aorta distal to the branch vessels 2218, 2220 (e.g., renal arteries) by between about 5% and about 30% as compared to normal flow. The implantable medical device 2200 may occlude the aorta distal to the branch vessels 2218, 2220 (*e.g.,* renal arteries) by between about 5% and about 30% to increase blood flow into branch vessels 2218, 2220 (*e.g.,* into the kidneys). The implantable medical device 2200 may be tailored to achieve between about 5% and about 30% increase of blood flow into the kidneys.

In certain instances, the implantable medical device 2200 is configured to induce stenosis of the aorta of the patient at least partially distal of the branch vessels 2218, 2220 (e.g., renal arteries) between about 40% and about 80% and alter blood flow into one or more of the branch vessels 2218, 2220 of the aorta (*e.g.,* one or both of the renal arteries) while maintaining a substantially unrestricted blood flow within the aorta proximal to one or more of the branch vessels 2218, 2220 of the aorta (*e.g.,* one or both of the renal arteries). In certain instances, the induced stenosis is between 20% and 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion.

In addition, the implantable medical device 2200 may be implanted into another vessel of the patient that leads into an organ. In these instances, the implantable medical device 2200 is configured to induce stenosis of the vessel into which the implantable medical device 2200 distal of location at which the implantable medical device 2200 is implanted between about 40% and about 80%. In addition, implanting the implantable medical device 300 in this manner alter blood flow into the organ that the vessel leads into while maintaining a substantially unrestricted blood flow within the vessel proximal to the location of implantation.

By adjusting the degree of hemodynamic alteration of renal perfusion, patient-specific adjustments to regulate blood pressure may be made. Adjusting the aortic flow resistance imparted by the implantable medical device 2200 may influence renal artery pressure and/or flow rate, which, in turn, can manifest as transient or long-lasting alterations in systemic blood pressure. The changes induced by the implantable medical device 2200, in renal-mediated blood pressure levels, may have therapeutic benefits in and of themselves. Likewise, changes induced by the implantable medical device 2200 in renal-mediated blood pressure levels may be used in combination with various blood pressure medications to optimize blood pressure management on an individualized basis.

In certain instances and as noted above, the implantable medical device 2200 may be implanted into another vessel of the patient that leads into an organ. In these instances, the implantable medical device 2200 is configured to induce stenosis of the vessel into which the implantable medical device 2200 distal of location at which the implantable medical device 2200 is implanted between about 40% and about 80%. In addition, implanting the implantable medical device 2200 in this manner alter blood flow into the organ that the vessel leads into while maintaining a substantially unrestricted blood flow within the vessel proximal to the location of implantation.

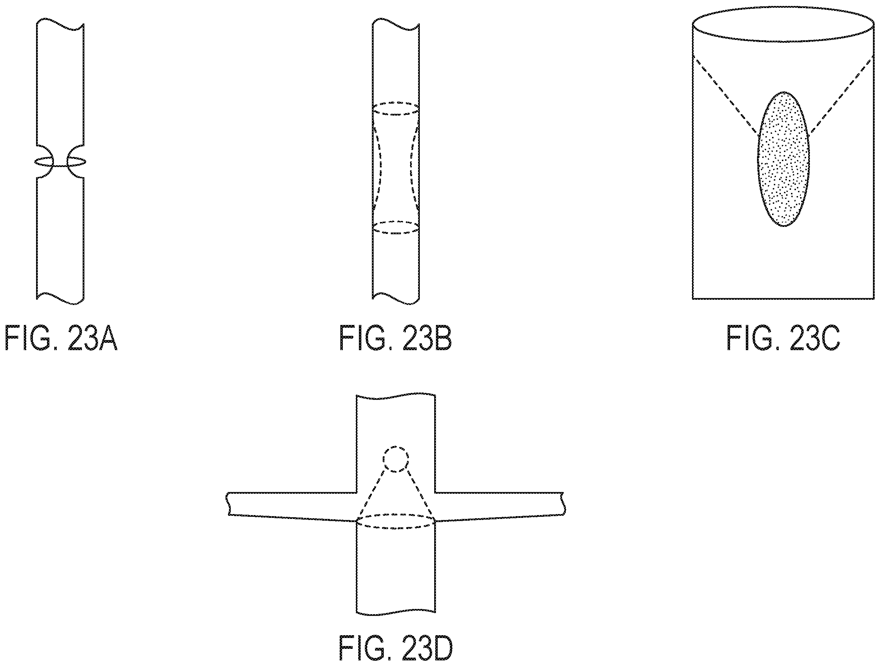

FIG. 23A-D show various example implantable medical devices (not according to claim 1). The implantable medical devices may be flow restriction cuff arranged about the aorta as shown in FIG. 23A, a stent-graft with a reduced diameter section as shown in FIG. 23B and FIG. 23D, or an implantable medical device having an interior restriction portion as shown in FIG. 23C.

In certain instances, patients with heart failure (such as late-stage heart failure) may have an elevated sympathetic nervous system state in part due to decreased cardiac output (blood pressure and flow in one of both of the kidneys). One compensatory output of this state is to generate a signal to attempt to preserve cardiac output, which puts further strain (myocardial oxygen demand) on the heart. Implantable medical devices discussed herein, and the methods that include the implantable medical devices, are directed toward increasing the pressure (mean or peak systolic) in the kidney to reduce stimulation of the neuro-hormonal response (*e.g.,* decrease in the sympathetic activation nervous system). The result of the reduced activation of the sympathetic nervous system, by way of the implantable medical device or methods that include the implantable medical device, may decrease resting heart rate and blood pressure.

Further and in certain instances, patients with heart failure (such as late-stage heart failure) may have activation of the Renin-Angiotensin-Aldosterone system (RAAS), in part due to decreased cardiac output resulting in impaired blood flow to the kidney. A consequence of the elevated RAAS is to generate a signal that stimulates adverse myocardial structural changes. Implantable medical devices discussed herein, and the methods that include the implantable medical devices, are directed toward increasing the pressure (mean or peak systolic) in the kidney to reduce stimulation of the RAAS. The result of the reduced activation of the RAAS, by way of the implantable medical device or methods that include the implantable medical device, may be a reduced sympathetic nervous system activation and attenuation of adverse cardiac remodeling.

Previous studies of implantable medical devices implanted in the aorta have been used to evaluate response of canines with induced heart failure (coronary microembolization resulting in ejection fraction of about 30%). Hemodynamic status observed in the test group relative to the control group indicated improved cardiac function and decreased sympathetic nervous system tone. For example, heart rate and mean arterial pressure decreased, while contractility increased relative to controls. These comparative outcomes were supported by positive shifts in biomarkers such as NT-proBNP and NGAL, relative to controls. As an example, animals with implant produced about 35% more urine with about 21% higher creatinine content, resulting in about 52% less increase in serum creatinine as a result of the diuretic challenge. These results show that an implantable medical device, such an implantable medical device directing blood into the kidneys or restricting blood flow within the aorta distal to the renal arteries, placed in the aorta may help decrease the symptoms of fluid overload and cardiac stress associated with heart failure. The devices are shown to increase blood pressure proximal to the stenosis and, in doing so, increase kidney perfusion pressure, thus increasing kidney perfusion. A secondary effect of this device is the reduction in the activation of the RAAS system. Effectiveness of the device was based on assessment of central hemodynamics, left ventricular (LV) function and renal function.

In addition, previous studies have found that induced stenosis has little effect on flow or pressure until it reaches about 40%, after which the impact is dependent on artery diameter and blood flow rate. Based on the above animal study, however, it has been discovered that there is a threshold above which the impact dramatically increases. The regime for stenosis, based on these results, is between about 40% - about 80%, and more particularly between about 50% - about 70%. Clinically, measurement of ankle pressure, Doppler ultrasound velocity, or other hemodynamic parameters in the lower limbs can be employed to optimize the magnitude of the induced stenosis while ensuring adequate limb perfusion.

In addition and in certain instances, the devices discussed above describe diametric restriction of the aorta or vena cava, however, a length of the restriction portion also may affect the amount of restriction in the aorta or vena cava. Thus, the length of the restriction portion and the diameter or circumference of the restriction portion may be varied to achieve a desired stenosis or restriction percentage.

Further, the devices discussed herein may implanted within vessels for treatment of an arteriovenous (AV) fistula. AV fistula formation may lead to a decrease in peripheral resistance within the vasculature. Implantation of the devices discussed herein at or adjacent to the AV fistula may increase flow resistance to a nominal level and counteract the decrease in peripheral resistance resulting from the AV fistula. In certain instances, the devices are implanted distal to the AV fistula, proximal to the AV fistula, or across the AV fistula.

Examples of synthetic polymers (which may be used as a graft component) include, but are not limited to, nylon, polyacrylamide, polycarbonate, polyformaldehyde, polymethylmethacrylate, polytetrafluoroethylene, polytrifluorochlorethylene, polyvinylchloride, polyurethane, elastomeric organosilicon polymers, polyethylene, polypropylene, polyurethane, polyglycolic acid, polyesters, polyamides, their mixtures, blends and copolymers are suitable as a graft material. In one embodiment, said graft is made from a class of polyesters such as polyethylene terephthalate including DACRON^{®} and MYLAR ^{®} and polyaramids such as KEVLAR^{®}, polyfluorocarbons such as polytetrafluoroethylene (PTFE) with and without copolymerized hexafluoropropylene (TEFLON^{®}. or GORE-TEX^{®}.), and porous or nonporous polyurethanes. In certain instances, the graft comprises expanded fluorocarbon polymers (especially PTFE) materials described in British. Pat. No. 1,355,373; 1,506,432; or 1,506,432 or in U.S. Pat. No. 3,953,566; 4,187,390; or 5,276,276. Included in the class of preferred fluoropolymers are polytetrafluoroethylene (PTFE), fluorinated ethylene propylene (FEP), copolymers of tetrafluoroethylene (TFE) and perfluoro(propyl vinyl ether) (PFA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinyfluoride (PVF). Especially preferred, because of its widespread use in vascular prostheses, is ePTFE. In certain instances, the graft comprises a combination of said materials listed above. In certain instances, the graft is substantially impermeable to bodily fluids. Said substantially impermeable graft can be made from materials that are substantially impermeable to bodily fluids or can be constructed from permeable materials treated or manufactured to be substantially impermeable to bodily fluids (e.g. by layering different types of materials described above or known in the art).

Additional examples of graft materials include, but are not limited to, vinylidinefluoride/hexafluoropropylene hexafluoropropylene (HFP), tetrafluoroethylene (TFE), vinylidenefluoride, 1-hydropentafluoropropylene, perfluoro(methyl vinyl ether), chlorotrifluoroethylene (CTFE), pentafluoropropene, trifluoroethylene, hexafluoroacetone, hexafluoroisobutylene, fluorinated poly(ethylene-co-propylene (FPEP), poly(hexafluoropropene) (PHFP), poly(chlorotrifluoroethylene) (PCTFE), poly(vinylidene fluoride (PVDF), poly(vinylidene fluoride-co-tetrafluoroethylene) (PVDF-TFE), poly(vinylidene fluoride-co-hexafluoropropene) (PVDF-HFP), poly(tetrafluoroethylene-co-hexafluoropropene) (PTFE-HFP), poly(tetrafluoroethylene-co-vinyl alcohol) (PTFE-VAL), poly(tetrafluoroethylene-co-vinyl acetate) (PTFE-VAC), poly(tetrafluoroethylene-co-propene) (PTFEP) poly(hexafluoropropene-co-vinyl alcohol) (PHFP-VAL), poly(ethylene-co-tetrafluoroethylene) (PETFE), poly(ethylene-co-hexafluoropropene) (PEHFP), poly(vinylidene fluoride-co-chlorotrifluoroe-thylene) (PVDF-CTFE), and combinations thereof, and additional polymers and copolymers described in U.S. Publication 2004/0063805. Additional polyfluorocopolymers include tetrafluoroethylene (TFE)/perfluoroalkylvinylether (PAVE). PAVE can be perfluoromethylvinylether (PMVE), perfluoroethylvinylether (PEVE), or perfluoropropylvinylether (PPVE), as essentially described in U.S. Publication 2006/0198866 and U.S. Pat. No. 7,049,380. Other polymers and copolymers include, polylactide, polycaprolacton-glycolide, polyorthoesters, polyanhydrides; poly-aminoacids; polysaccharides; polyphosphazenes; poly(ether-ester) copolymers, e.g., PEO-PLLA, or blends thereof, polydimethyl-siolxane; poly(ethylene-vingylacetate); acrylate based polymers or copolymers, e.g., poly(hydroxyethyl methylmethacrylate, polyvinyl pyrrolidinone; fluorinated polymers such as polytetrafluoroethylene; cellulose esters and any polymer and copolymers described in U.S. Publication 2004/0063805.

The graft components, as discussed herein, may be attached to the self-expanding stent elements by using a coupling member that is generally a flat ribbon or tape having at least one generally flat surface. In certain instances, the tape member is made from expanded PTFE (ePTFE) coated with an adhesive. The adhesive may be a thermoplastic adhesive. In certain instances, the thermoplastic adhesive may be fluorinated ethylene propylene (FEP). More specifically, an FEP-coated side of the ePTFE may face toward and contacts an exterior surface of the self-expanding stent and graft component, thus attaching the self-expanding stent to the graft component. Materials and method of attaching a stent to the graft is discussed in U.S. Pat. No. 6,042,602 to Martin.

The stent elements discussed herein can be fabricated from a variety of biocompatible materials. These materials may include 316L stainless steel, cobalt-chromium-nickel-molybdenum-iron alloy ("cobalt-chromium"), other cobalt alloys such as L605, tantalum, nickel-titanium alloys (e.g., Nitinol), or other biocompatible metals. In certain instances, as discussed in detail above, the stent (and graft) may be self-expanding. The prosthesis may be balloon expandable

A variety of materials variously metallic, super elastic alloys, such as Nitinol, are suitable for use in these stents. Primary requirements of the materials are that they be suitably springy even when fashioned into very thin sheets or small diameter wires. Various stainless steels which have been physically, chemically, and otherwise treated to produce high springiness are suitable as are other metal alloys such as cobalt chrome alloys (*e.g*., ELGILOY^{®}), platinum/tungsten alloys, and especially the nickel-titanium alloys (*e.g*., Nitinol).

## Claims

1. An implantable medical device (800, 900) for altering blood flow in a vessel of a patient, the device comprising:
a stent element (918) and a graft component (920) attached to at least a portion of the stent element, the stent element and the graft component having:
an adjustable portion (800b, 900b) configured to alter an amount of directed blood flow by a restricting portion (800a, 900a), the restricting portion (800a, 900a) being configured to direct flow into one or more side branches off the vessel, wherein the restricting portion (800a, 900a) forms a channel through the adjustable portion (800b, 900b) to allow fluid flow therethrough, wherein the device incrementally decreases in diameter from the adjustable portion (800b, 900b) to the restricting portion (800a, 900b);
**characterized in that**
the device is adjustable between a relaxed configuration and a lengthened, stretched configuration so that fluid flow through the device can be adjusted; and **in that**
the device has a fluid inlet (810, 910) and a fluid outlet (812, 912),
wherein as the device (800, 900) lengthens from the relaxed configuration to the stretched configuration, the fluid outlet (812, 912) changes from the first outlet diameter (d_{O1}) to a second, smaller outlet diameter (d_{O2}).

2. The implantable medical device (800, 900) of claim 1, wherein the device is tapered.

3. The implantable medical device (800, 900) of claim 1, wherein the adjustable portion (800b, 900b) is conical or tapered.

4. The implantable medical device (800) of claim 1, comprising an inner structure and an outer structure, wherein the inner structure is connected to the restricting portion (800a) and extends through the outer structure.

5. The implantable medical device (800) of claim 1, wherein the inner and outer structures are tapered in the extended configuration.

6. The implantable medical device (800, 900) of any preceding claim, wherein the adjustable portion (800b, 900b) includes a multi-layer stent-structure having a membrane and the membrane is configured to restrict blood flow in response to increased blood pressure during the cardiac output phase.

7. The implantable device (900) of claim 1, wherein the device comprises a series of stacked ring structures (918), or a spiral ring structure (918).

8. The implantable device (900) of claim 7, wherein the spiral ring structure (918) is coupled to the graft material (920).

9. The implantable device (900) of claim 8, wherein the stacked ring structures (918) are connected to one another via the graft material (920).

## Patentansprüche

1. Implantierbare medizinische Vorrichtung (800, 900) zum Ändern einer Blutströmung in einem Gefäß eines Patienten, wobei die Vorrichtung Folgendes umfasst:
ein Stent-Element (918) und eine Graft-Komponente (920), die an mindestens einem Abschnitt des Stent-Elements befestigt ist, wobei das Stent-Element und die Graft-Komponente Folgendes aufweisen:
einen einstellbaren Abschnitt (800b, 900b), der dazu konfiguriert ist, eine Menge an geleiteter Blutströmung durch einen einschränkenden Abschnitt (800a, 900a) zu ändern, wobei der einschränkende Abschnitt (800a, 900a) dazu konfiguriert ist, eine Strömung in einen oder mehrere Seitenäste dem Gefäß weg zu leiten, wobei der einschränkende Abschnitt (800a, 900a) einen Kanal durch den einstellbaren Abschnitt (800b, 900b) ausbildet, um eine Fluidströmung durch diesen zu ermöglichen,
wobei sich der Durchmesser der Vorrichtung von dem einstellbaren Abschnitt (800b, 900b) zu dem einschränkenden Abschnitt (800a, 900b) schrittweise verringert;
**dadurch gekennzeichnet, dass** die Vorrichtung zwischen einer entspannten Konfiguration und einer verlängerten, gestreckten Konfiguration einstellbar ist, sodass die Fluidströmung durch die Vorrichtung eingestellt werden kann; und dadurch, dass
die Vorrichtung einen Fluideinlass (810, 910) und einen Fluidauslass (812, 912) aufweist,
wobei sich der Fluidauslass (812, 912) von dem ersten Auslassdurchmesser (d_{O1}) zu einem zweiten, kleineren Auslassdurchmesser (d_{O2}) ändert, wenn sich die Vorrichtung (800, 900) von der entspannten Konfiguration zu der gestreckten Konfiguration verlängert.

2. Implantierbare medizinische Vorrichtung (800, 900) nach Anspruch 1, wobei die Vorrichtung sich verjüngt.

3. Implantierbare medizinische Vorrichtung (800, 900) nach Anspruch 1, wobei der einstellbare Abschnitt (800b, 900b) konisch ist oder sich verjüngt.

4. Implantierbare medizinische Vorrichtung (800) nach Anspruch 1, umfassend eine Innenstruktur und eine Außenstruktur, wobei die Innenstruktur mit dem einschränkenden Abschnitt (800a) verbunden ist und sich durch die Außenstruktur erstreckt.

5. Implantierbare medizinische Vorrichtung (800) nach Anspruch 1, wobei sich die Innen- und die Außenstruktur in der erweiterten Konfiguration verjüngen.

6. Implantierbare medizinische Vorrichtung (800, 900) nach einem vorhergehenden Anspruch, wobei der einstellbare Abschnitt (800b, 900b) eine mehrschichtige Stentstruktur mit einer Membran enthält und die Membran dazu konfiguriert ist, eine Blutströmung als Reaktion auf erhöhten Blutdruck während der Herzauswurfphase einzuschränken.

7. Implantierbare Vorrichtung (900) nach Anspruch 1, wobei die Vorrichtung eine Reihe von gestapelten Ringstrukturen (918) oder eine Spiralringstruktur (918) umfasst.

8. Implantierbare Vorrichtung (900) nach Anspruch 7, wobei die Spiralringstruktur (918) mit dem Graft-Material (920) gekoppelt ist.

9. Implantierbare Vorrichtung (900) nach Anspruch 8, wobei die gestapelten Ringstrukturen (918) über das Graft-Material (920) miteinander verbunden sind.

## Revendications

1. Dispositif médical implantable (800, 900) permettant de modifier le flux de sang dans un vaisseau d'un patient, le dispositif comprenant :
un élément d'endoprothèse (918) et un composant de greffe (920) fixé à au moins une partie de l'élément d'endoprothèse, l'élément d'endoprothèse et le composant de greffe comportant :
une partie réglable (800b, 900b) conçue pour modifier une quantité de flux sanguin dirigé par une partie de restriction (800a, 900a), la partie de restriction (800a, 900a) étant conçue pour diriger le flux dans une ou plusieurs ramifications latérales du vaisseau, ladite partie de restriction (800a, 900a) formant un canal à travers la partie réglable (800b, 900b) de manière à permettre le flux de fluide à travers celle-ci,
ledit dispositif diminuant progressivement en diamètre de la partie réglable (800b, 900b) à la partie de restriction (800a, 900b) ;
**caractérisé en ce que** le dispositif est réglable entre une configuration détendue et une configuration allongée et étirée de sorte que le flux de fluide à travers le dispositif puisse être réglé ; et **en ce que** le dispositif comporte une entrée de fluide (810, 910) et une sortie de fluide (812, 912),
à mesure que le dispositif (800, 900) s'allonge de la configuration détendue à la configuration étirée, ladite sortie de fluide (812, 912) passant du premier diamètre de sortie (d_{O1}) à un second diamètre de sortie plus petit (d_{O2}).

2. Dispositif médical implantable (800, 900) selon la revendication 1, ledit dispositif étant effilé.

3. Dispositif médical implantable (800, 900) selon la revendication 1, ladite partie réglable (800b, 900b) étant conique ou effilée.

4. Dispositif médical implantable (800) selon la revendication 1, comprenant une structure interne et une structure externe, ladite structure interne étant reliée à la partie de restriction (800a) et s'étendant à travers la structure externe.

5. Dispositif médical implantable (800) selon la revendication 1, lesdites structures interne et externe étant effilées dans la configuration étendue.

6. Dispositif médical implantable (800, 900) selon l'une quelconque des revendications précédentes, ladite partie réglable (800b, 900b) comprenant une structure d'endoprothèse multicouche comportant une membrane et ladite membrane étant conçue pour restreindre le flux sanguin en réponse à une augmentation de la pression artérielle pendant la phase de débit cardiaque.

7. Dispositif implantable (900) selon la revendication 1, ledit dispositif comprenant une série de structures annulaires empilées (918), ou une structure annulaire en spirale (918).

8. Dispositif implantable (900) selon la revendication 7, ladite structure annulaire en spirale (918) étant couplée au matériau de greffe (920).

9. Dispositif implantable (900) selon la revendication 8, lesdites structures annulaires empilées (918) étant reliées les unes aux autres par l'intermédiaire du matériau de greffe (920).
